# EUROPEAN PATENT SPECIFICATION

(11) **EP 3 965 828 B1**
(45) Date of publication and mention of the grant of the patent: **21.06.2023**
(21) Application number: 20724828.7
(22) Date of filing: 07.05.2020
(51) Int. Cl.: A61K 47/69, A61K 47/64, C12N 15/11, C12N 15/88, C07K 14/705, A61P 43/00, C07K 14/47

(54) **EXOSOME COMPRISING STABILIZED RNA THERAPEUTICS**
EXOSOM MIT STABILISIERTEN RNA-THERAPEUTIKA
EXOSOMES COMPRENANT DES AGENTS THÉRAPEUTIQUES À BASE D'ARN STABILISÉS

(30) Priority: 08.05.2019 GB 201906482
(43) Date of publication of application: 16.03.2022
(73) Proprietor: Evox Therapeutics Ltd, Oxford OX4 4HG (GB)
(72) Inventor: CASTILLA LLORENTE, Virginia, Oxford Oxfordshire OX4 4HG (GB); LIANG, Xiuming, 14156 Stockholm (SE); ZICKLER, Antje, 11346 Stockholm (SE); DE LUCA, Mariacristina, Oxford Oxfordshire OX4 4EP (GB); ERRICHELLI, Lorenzo, Oxford Oxfordshire OX1 4LZ (GB); SMITH, Christopher, Oxford Oxfordshire OX4 4EP (GB); TSALIC, Ran, Abingdon Oxfordshire OX14 3UR (GB)
(74) Representative: Cooley (UK) LLP
(86) International application number: PCT/EP2020/062791
(87) International publication number: WO 2020/225392

(56) References cited:
- WO-A1-2019/092145
- Lysangela R. Alves ET AL: "Extracellular vesicle-mediated RNA release in Histoplasma 2 capsulatum", bioRxiv, 7 March 2019 (2019-03-07), XP055718329, DOI: 10.1101/570291 Retrieved from the Internet: URL:https://www.biorxiv.org/content/10.110 1/570291v1.full.pdf [retrieved on 2020-07-27]
- MICHELLE E. HUNG ET AL: "A platform for actively loading cargo RNA to elucidate limiting steps in EV-mediated delivery", JOURNAL OF EXTRACELLULAR VESICLES, vol. 5, no. 1, 1 January 2016 (2016-01-01) , page 31027, XP055711976, DOI: 10.3402/jev.v5.31027
- DHRUVITKUMAR S. SUTARIA ET AL: "Low active loading of cargo into engineered extracellular vesicles results in inefficient miRNA mimic delivery", JOURNAL OF EXTRACELLULAR VESICLES, vol. 6, no. 1, 14 June 2017 (2017-06-14), page 1333882, XP055611131, DOI: 10.1080/20013078.2017.1333882
- DHRUVITKUMAR S. SUTARIA ET AL: "Achieving the Promise of Therapeutic Extracellular Vesicles: The Devil is in Details of Therapeutic Loading", PHARMACEUTICAL RESEARCH, vol. 34, no. 5, 17 March 2017 (2017-03-17) , pages 1053-1066, XP055543423, US ISSN: 0724-8741, DOI: 10.1007/s11095-017-2123-5

## Description

### Technical Field

The present invention relates to extracellular vesicle (EV) therapeutics, wherein the EVs comprise endogenously loaded, stabilized nucleic acids, polypeptide and polynucleotide constructs for endogenously loading such stabilized nucleic acids into EVs, methods of producing said EVs, cells engineered to produce said EVs and use of said EVs in medicine.

### Background to the Invention

Nucleic acid (NA) based therapeutics are approaching extensive clinical utility at a rapid pace. Gene replacement therapies, mRNA-based therapies, short oligonucleotide and siRNA based therapeutics are just some examples within the plethora of modalities within the RNA therapeutics landscape. Naked nucleic acids, typically mRNA, are difficult to deliver *in vivo* due to rapid clearance, nuclease activity, lack of organ-specific distribution, and low efficacy of cellular uptake, meaning that specialized delivery vehicles are usually obligatory as a means of achieving bioactive delivery. This is especially the case for non-hepatic targets and for highmolecular weight RNA therapeutics such as mRNA therapeutics.

Extracellular vesicles (such as exosomes) are typically nanometer-sized vesicles produced by most cell types and function as the body's natural transport system for proteins, nucleic acids, peptides, lipids, and various other molecules between cells. RNA-containing EVs have a number of potential therapeutic uses and EVs are already being investigated as delivery vehicles for mRNA delivery, siRNA therapeutics and delivery of other short nucleic acids drugs in various settings. WO2010/119256 represents the foundational invention in the field of nucleic acid delivery using exosomes and said invention teaches the utility of exosomes for delivery of several types of nucleic acid cargo. PCT/GB2017/051479 teaches improved methods for endogenous loading of various types of RNA therapeutics with the aid of RNA-binding proteins, which drag RNA of interest into EVs forming within parental cells. US14/502,494 represents another method for utilizing RNA-binding proteins to load certain RNA species, utilizing the so called The Targeted And Modular Exosome Loading (TAMEL) system.

However, despite these advances, there is still significant room in the art for improved loading of large and small NA cargos into EVs in a specific and efficient manner. Furthermore, the actual bioactive delivery into target cells is a key aspect of any delivery system and the prior art has room for improvement also in this regard. Further still all NA delivery technologies suffer from the relatively short and variable half-life of nucleic acids, particularly mRNA, once delivered to recipient cells as well as the NA having a short half-life during production and storage of the NA therapy.

The TAMEL loading system described in US14/502,494 and the corresponding literature reference (Hung and Leonard, Journal of Extracellular Vesicles 2016, 5: 31027) has a number of disadvantages which the present invention attempts to overcome. The major disadvantage of the TAMEL system is that it is unable to achieve functional delivery of mRNA, i.e. the mRNA loaded into the exosomes by the TAMEL system is not translated by the cells when cells are exposed to these exosomes. The present application achieves bioactive delivery of the cargo NA.

Tutucci et al (An improved MS2 system for accurate reporting of the mRNA life cycle. Nat Methods. 2018 Jan; 15(1): 81-89) discusses the use of the MS2 protein for studying RNA localization and lifecycle and indicates that the MS2 protein as used by the TAMEL system binds with very high affinity to the RNA. This tight binding is reported by this paper to be problematic for the study of mRNA regulation. We surmise that in the context of EV loading this high affinity binding is again problematic because the tight binding of the target RNA by MS2 means that any RNA that is loaded will not be released. Preventing release of mRNA will prevent normal translation and this would explain why no translation of the mRNA is observed in US14/502,494.

The TAMEL system does not appear to load all exosomes with nucleic acids and those that are loaded are loaded with very small numbers of nucleic acids (disadvantages of this variable and low level of loading are discussed in detail below). These variable and low levels of loading combined with the fact that what little nucleic acid that is loaded is then unlikely to be released and therefore not bioactive and what is released having only a short lifespan due to instability of the native mRNA means that the TAMEL system has many disadvantages. The TAMEL system is not suitable for loading and delivery of clinically relevant quantities of bioactive nucleic acids. The present invention overcomes these significant disadvantages.

Another disadvantage of the TAMEL system is that it employs bacteriophage proteins which may illicit unwanted immunological responses when the EVs produced are delivered to patients. The present invention overcomes this disadvantage too.

### Summary of the Invention

It is hence an object of the present invention to overcome the above-identified problems associated with loading and subsequent EV-mediated delivery of bioactive NA-based therapeutics, and to satisfy the existing needs within the art, for instance to enable reaching the right intracellular compartment with an intact and highly stable NA therapeutic agent. The present invention achieves this by utilizing novel EV engineering technology to load and release stabilized NA (nucleic acid) cargo, preferably mRNA cargo.

This is achieved by advanced engineering of polypeptide and polynucleotide constructs as well as engineering of producer cells to ensure not only highly efficient loading into EVs, but a high degree of stability of the NA when produced, loaded and delivered and also effective release of the NA in question.

In a first aspect the present invention relates to an extracellular vesicle (EV) comprising at least one polyA binding protein (PABP) or a fragment or domain thereof and at least one NA cargo molecule comprising a contiguous stretch of adenine nucleotides (i.e. a polyA region or tail).

Advantageously the EV of the invention comprises at least one fusion polypeptide comprising at least one nucleic acid (NA)-binding domain and at least one exosomal polypeptide, wherein optionally PABP is comprised in the fusion polypeptide. In this embodiment the NA is transported into the EV by the fusion protein. Advantageously the EV may further comprise at least one targeting moiety.

The present invention also relates to a population of EVs according to the invention. Advantageously the population of EVs has at least one NA cargo molecule per EV and/or up to 95% of the population of EVs comprise a NA cargo molecule.

The present invention also relates to methods for producing EVs according to the invention, the method comprising: introducing into an EV-producing cell at least one polynucleotide construct encoding for a PABP protein or a fragment or domain thereof; and introducing into the same EV-producing cell at least one polynucleotide construct encoding at least one NA cargo molecule comprising a polyA region; and expressing in said EV-producing cell PABP and the NA cargo molecule, thereby generating said EVs. Advantageously the method further comprises: introducing into the EV-producing cell at least one polynucleotide construct encoding for a fusion polypeptide comprising at least one NA-binding domain and at least one exosomal polypeptide and optionally a polynucleotide construct encoding for a translation initiation factor; expressing in the EV-producing cell the at least one polypeptide construct encoded for by the polynucleotide constructs thereby generating said EVs. In other advantageous aspects the PAPB protein or fragment or domain thereof forms part of the fusion polypeptide comprising at least one NA-binding domain and at least one exosomal polypeptide in the method of the present invention.

In yet another aspect the present invention relates to an *in vitro* method for intracellular delivery of at least one NA cargo molecule, comprising contacting a target cell with at least one EV according to the invention and/or at least one population of EVs according to the invention
The present invention also relates to a pharmaceutical composition comprising (i) at least one EV of the invention, and/or (ii) at least one population of EVs of the invention, and a pharmaceutically acceptable excipient or carrier.

In another aspect the present invention also relates to at least one: EV, population of EVs and/or pharmaceutical composition according to the present invention, for use in medicine.

### Brief Description of the Figures

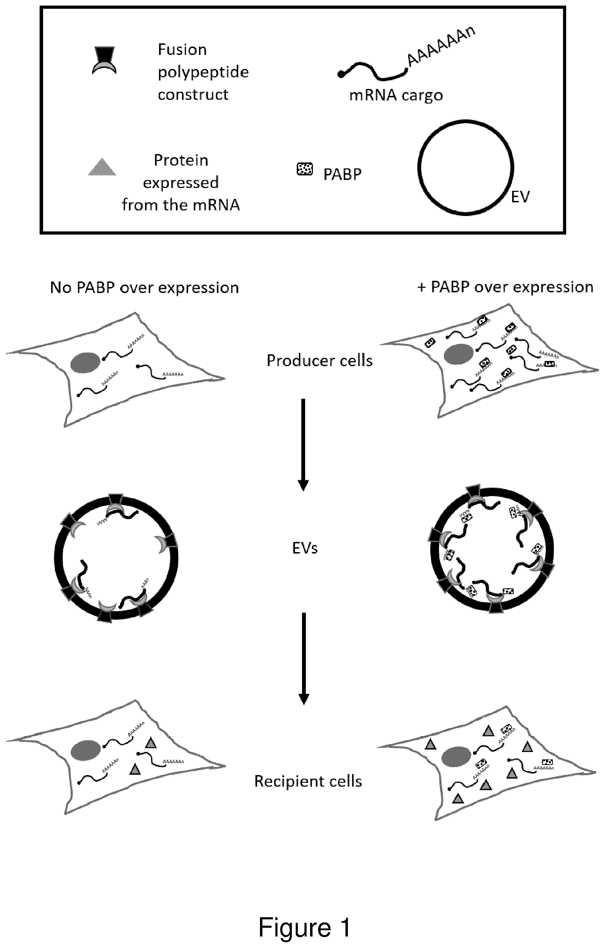

Figure 1: Schematic illustration of producer cells expressing the fusion protein construct with and without the overexpression of PABP, EVs produced by those producer cells loaded with NA cargo molecules (with and without PABP bound to and stabilizing the polyA segment of the NA cargo) using the fusion polypeptide constructs as per the present invention as well as recipient cells to which said EVs have been delivered and the protein expressed from the NA cargo.

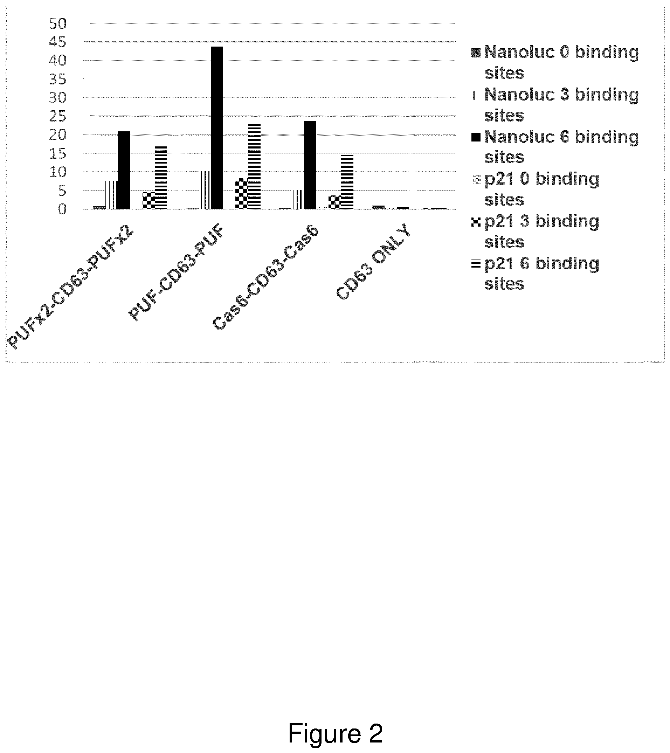

Figure 2: Graph showing loading, into MSC-derived EVs, of NA cargo molecules encoding NanoLuc (RTM) and p21, using fusion polypeptide constructs comprising CD63 as the exosomal polypeptide and PUF (in this case the NA-binding PUF domain is obtained from the human PUM1 protein) or Cas6 as the NA-binding domains. The experiment also included varying numbers of binding sites for the NA-binding domains, namely 0, 3 and 6 binding sites. Expression of only the exosomal polypeptide CD63 did not result in loading of any mRNA into the EVs (right). Expression of fusion polypeptides comprising PUF (left set of columns: two PUFs domain flanking CD63 both N terminally and C terminally, i.e. 4 PUF constructs in total) (second set of columns from left: one PUF domain flanking CD63 both N terminally and C terminally) and mutated Cas6 (second from right) did result in significant mRNA loading of both NanoLuc (RTM) and p21 mRNAs upon expression in the EV source cells. The loading of NanoLuc (RTM) was overall more efficient than the loading of p21, with up to around 45 copies of mRNA per EV.

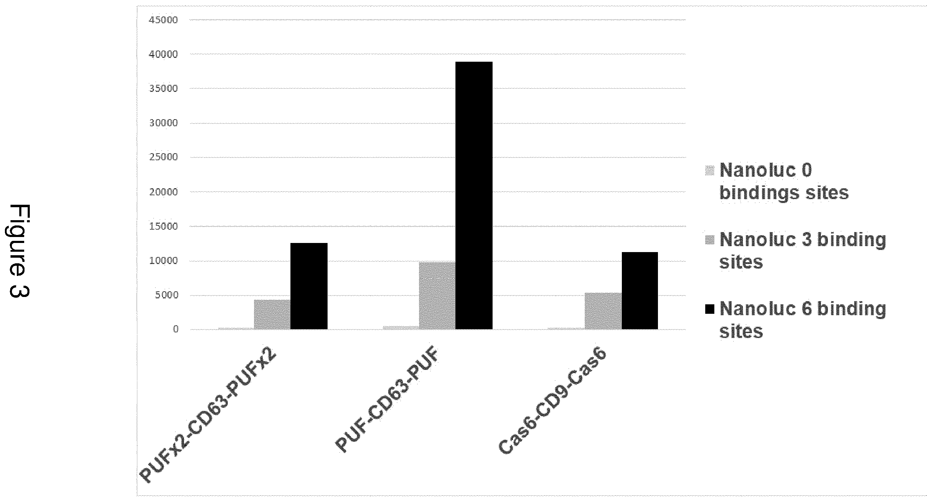

Figure 3: Expression of NanoLuc (RTM) as a reporter system in target HeLa cells after HEK EV-mediated delivery of a NanoLuc (RTM) mRNA. The NanoLuc (RTM) mRNA cargo molecule was engineered to comprise 0, 3, or 6 binding sites for the NA-binding domains comprising the fusion polypeptide constructs, in this case PUFx2-CD63-PUFx2 (two PUF NA-binding polypeptides inserted both N terminally and C terminally of the exosomal polypeptide CD63), PUF-CD63-PUF, and Cas6-CD9-Cas6. The Y axis shows relative light (luminescence) units (RLU) normalized over µg of protein, indicating enhanced delivery and/or translation with increasing numbers of binding sites. Human PUM1 and also an NA-binding PUF obtained from Pumilio protein of D. melanogaster was evaluated in this assay.

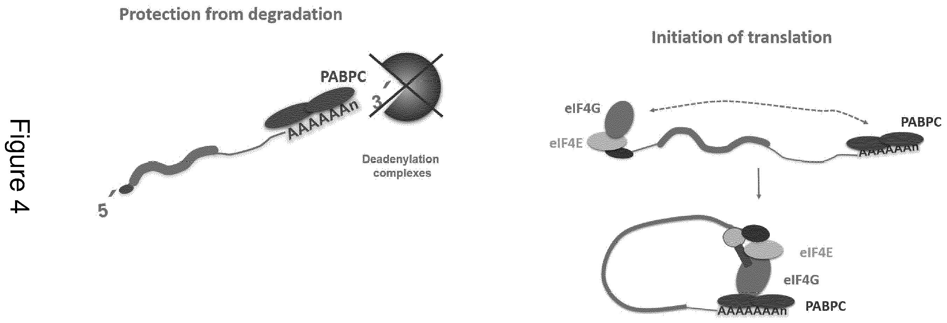

Figure 4: Schematic showing the main cellular functions of PABP. Schematic illustration of the main functions of PABP. (A) PABP interacts with the polyA tail of mRNA molecules protecting them from deadenylation and subsequent degradation. (B) PABP interacts with the polyA tail of mRNA molecules, as well as with elF4G, a translation initiation factor, leading to the formation of the translation initiation complex.

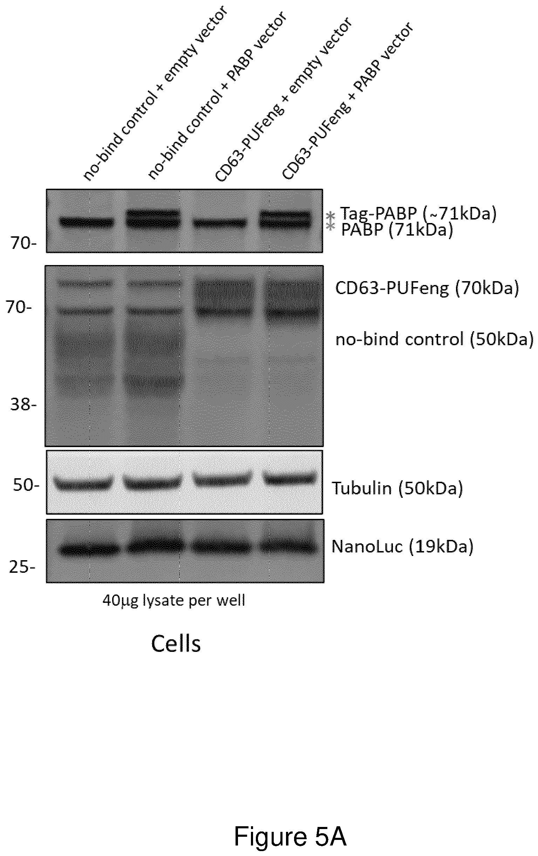

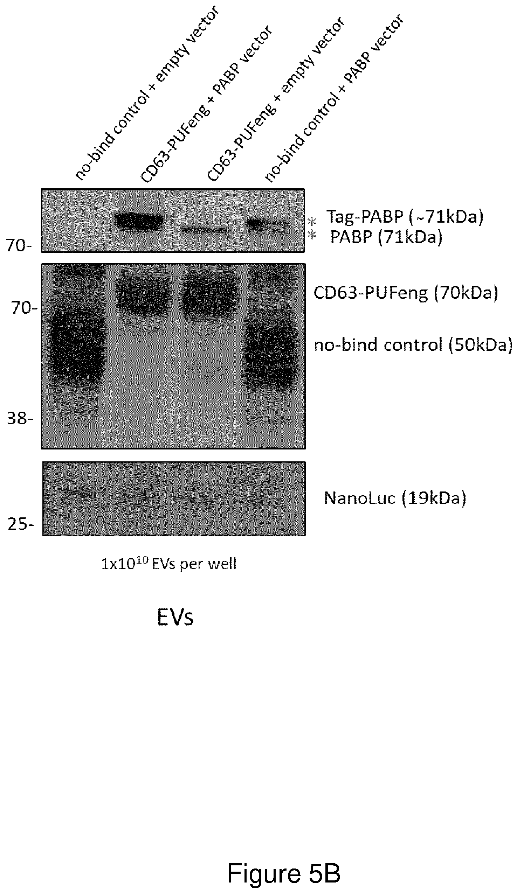

Figure 5A and 5B: Western blots showing transiently transfected HA-PABP and CD63-PUFeng are expressed in HEK293T cells stably expressing Nanoluc mRNA and both proteins are also present in extracellular vesicles purified from the conditioned media.

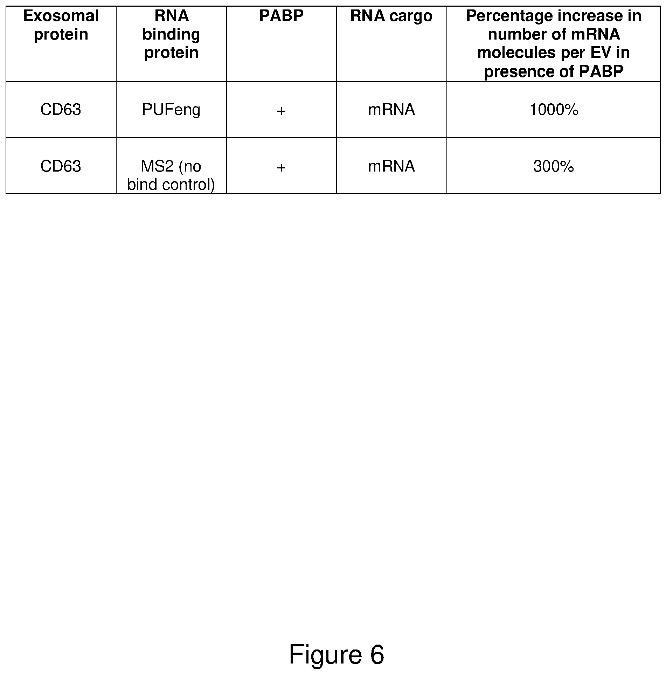

Figure 6: Table showing the percentage increase in mRNA molecules per EV by overexpression of PABP in producer cells to increase the number of molecules loaded into EVs.

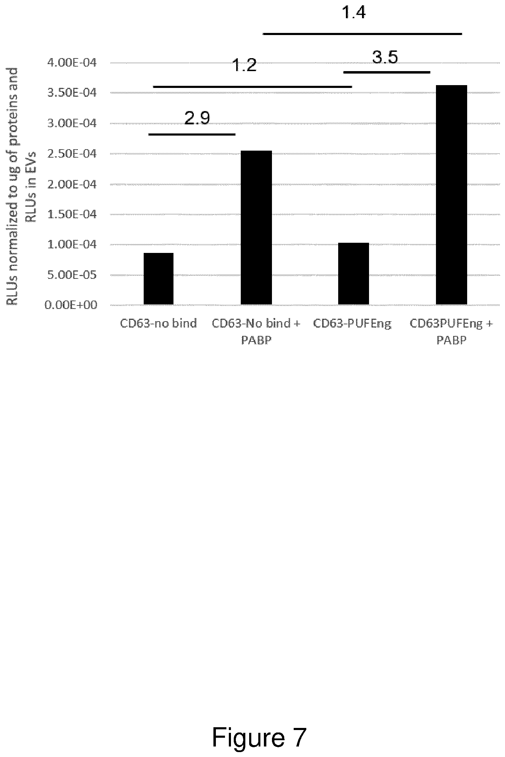

Figure 7: Graph showing the number of Nanoluc mRNA molecules delivered to Huh7 recipient cells is higher in the presence of PABP and also leads to an increase in the RLUs detected in the cell lysates as a result of an increase of translation of the delivered mRNA molecules.

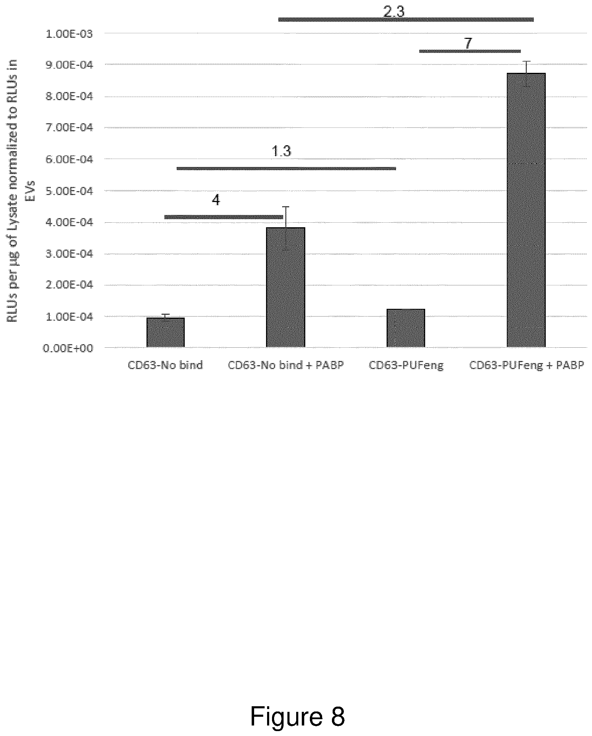

Figure 8: Graph showing repeat of uptake experiment shown in Figure 7 performed at 10x dilution. Dilution allows clearer distinction of the levels of mRNA expression. The number of Nanoluc mRNA molecules delivered to Huh7 recipient cells is higher in the presence of PABP and also leads to an increase (7 fold) in the RLUs detected in the cell lysates as a result of an increase in translation of the delivered mRNA molecules.

### Detailed Description of the Invention

The present invention relates to improved stability, loading, controlled release, and thus enhanced efficacy of EV-delivered NA therapeutics, using novel engineering approaches for introducing, stabilizing and delivering NA cargo in a bioactive fashion into target cells in vitro and/or in vivo.

For convenience and clarity, certain terms employed herein are collected and described below. Unless otherwise defined, all technical and scientific terms used herein have the same meaning as commonly understood by one of ordinary skill in the art to which this invention belongs.

Where features, aspects, embodiments, or alternatives of the present invention are described in terms of Markush groups, a person skilled in the art will recognize that the invention is also thereby described in terms of any individual member or subgroup of members of the Markush group. The person skilled in the art will further recognize that the invention is also thereby described in terms of any combination of individual members or subgroups of members of Markush groups. Additionally, it should be noted that embodiments and features described in connection with one of the aspects and/or embodiments of the present invention also apply mutatis mutandis to all the other aspects and/or embodiments of the invention. For example, the PABP and transcription initiation factor proteins and the fusion polypeptides described herein in connection with the EVs are to be understood to be disclosed, relevant, and compatible with all other aspects, teachings and embodiments herein, for instance aspects and/or embodiments relating to the methods for producing or the EVs, or relating to the corresponding polynucleotide constructs described herein or the engineered EV-producing cells from which the EVs derive. Furthermore, certain embodiments described in connection with certain aspects, for instance the administration routes of the EVs comprising the NA drug cargo molecule and optionally the fusion polypeptides, as described in relation to aspects pertaining to treating certain medical indications, may naturally also be relevant in connection with other aspects and/or embodiment such as those pertaining to the pharmaceutical compositions comprising such EVs. Furthermore, all polypeptides and proteins identified herein can be freely combined in fusion proteins using conventional strategies for fusing polypeptides. As a non-limiting example, NA-binding domains (which are of polypeptide origins) described herein may be freely combined in any combination with one or more exosomal polypeptides, optionally combined with all other polypeptide domains, regions, sequences, peptides, groups herein, e.g. any multimerization domains, release domains, and/or targeting peptides. Also, exosomal polypeptides and/or NA-binding domains may be combined with each other to generate constructs comprising more than one exosomal polypeptide and/or more than one NA-binding domain. Furthermore, PABP and/or transcription initiation factor protein may be included in such fusion proteins, to for instance in a non-limiting example create a fusion protein between an exosomal protein, an NA-binding domain and PABP, optionally with the additional including of a transcription initiation factor. Moreover, any and all features (for instance any and all members of a Markush group) can be freely combined with any and all other features (for instance any and all members of any other Markush group), e.g. any NA-binding domain (and/or any NA-binding protein from which such NA-binding domains are typically obtained) may be combined with any exosomal polypeptide. Furthermore, when teachings herein refer to EVs in singular and/or to EVs as discrete natural nanoparticle-like vesicles it should be understood that all such teachings are equally relevant for and applicable to a plurality of EVs and populations of EVs. As a general remark, the NA-binding domains, the exosomal polypeptides, the EV-producing cell sources, the additional domains and peptides, the NA cargo molecule, and all other aspects, embodiments, and alternatives in accordance with the present invention may be freely combined in any and all possible combinations without deviating from the scope and the gist of the invention. Furthermore, any polypeptide or polynucleotide or any polypeptide or polynucleotide sequences (amino acid sequences or nucleotide sequences, respectively) of the present invention may deviate considerably from the original polypeptides, polynucleotides and sequences as long as any given molecule retains the ability to carry out the desired technical effect associated therewith. As long as their biological properties are maintained the polypeptide and/or polynucleotide sequences according to the present application may deviate with as much as 50% (calculated using for instance BLAST or ClustalW) as compared to the native sequence, although a sequence identity or similarity that is as high as possible is preferable (for instance 60%, 70%, 80%, or e.g. 90% or higher). Standard methods in the art may be used to determine homology. For example, the UWGCG Package provides the BESTFIT program which can be used to calculate homology, for example used on its default settings (Devereux et al (1984) Nucleic Acids Research 12, p 387-395). The PILEUP and BLAST algorithms can be used to calculate homology or line up sequences (such as identifying equivalent residues or corresponding sequences (typically on their default settings)), for example as described in Altschul S. F. (1993) J Mol Evol 36:290-300; Altschul, S. F et al (1990) J Mol Biol 215:403-10. The combination (fusion) of e.g. several polypeptides implies that certain segments of the respective polypeptides may be replaced and/or modified and/or that the sequences may be interrupted by insertion of other amino acid stretches, meaning that the deviation from the native sequence may be considerable as long as the key properties (e.g. NA-binding, trafficking into EVs, targeting capabilities, etc.) are conserved. Similar reasoning thus naturally applies to the polynucleotide sequences encoding for such polypeptides. Any accession numbers or SEQ ID NOs mentioned herein in connection with peptides, polypeptides and proteins shall only be seen as examples and for information only, and all peptides, polypeptides and proteins shall be given their ordinary meaning as the skilled person would understand them. Thus, as above-mentioned, the skilled person will also understand that the present invention encompasses not merely the specific SEQ ID NOs and/or accession numbers referred to herein but also variants and derivatives thereof. All accession numbers referred to herein are UniProtKB accession numbers, and all proteins, polypeptides, peptides, nucleotides and polynucleotides mentioned herein are to be construed according to their conventional meaning as understood by a skilled person.

The terms "extracellular vesicle" or "EV" or "exosome" or "genetically modified/genetically engineered exosome" or "modified exosome" are used interchangeably herein and shall be understood to relate to any type of vesicle that is obtainable from a cell in any form, for instance a microvesicle (e.g. any vesicle shed from the plasma membrane of a cell), an exosome (e.g. any vesicle derived from the endosomal, lysosomal and/or endo-lysosomal pathway), an apoptotic body, ARMMs (arrestin domain containing protein 1 [ARRDC1]-mediated microvesicles), a microparticle and other vesicular structures, etc. The terms "genetically modified" and "genetically engineered" EV indicates that the EV is derived from a genetically modified/engineered cell usually comprising a recombinant or exogenous NA and/or protein product which is incorporated into the EVs produced by those cells. The term "modified EV" indicates that the vesicle has been modified either using genetic or chemical approaches, for instance via genetic engineering of the EV-producing cell or via e.g. chemical conjugation, for instance to attach moieties to the exosome surface. The sizes of EVs may vary considerably but an EV typically has a nano-sized hydrodynamic diameter, i.e. a diameter below 1000 nm. Clearly, EVs may be derived from any cell type, in vivo, ex vivo, and in vitro. Preferred EVs include exosomes and microvesicles, but other EVs may also be advantageous in various circumstances. Furthermore, said terms shall also be understood to relate to extracellular vesicle mimics, cell membrane-based vesicles obtained through for instance membrane extrusion, sonication, or other techniques, etc. It will be clear to the skilled artisan that when describing medical and scientific uses and applications of the EVs, the present invention normally relates to a plurality of EVs, i.e. a population of EVs which may comprise thousands, millions, billions or even trillions of EVs. As can be seen from the experimental section below, EVs may be present in concentrations such as 10^{5,} 10⁸, 10¹⁰, 10¹¹, 10¹², 10¹³, 10¹⁴, 10¹⁵, 10¹⁸, 10²⁵ , 10³⁰ EVs (often termed "particles") per unit of volume (for instance per ml), or any other number larger, smaller or anywhere in between. In the same vein, the term "population", which may e.g. relate to an EV comprising an NA cargo molecule such as an mRNA or an EV comprising a certain fusion polypeptide between an exosomal polypeptide and an NA-binding domain which in turn may be binding an NA cargo molecule of interest, shall be understood to encompass a plurality of entities constituting such a population. In other words, individual EVs when present in a plurality constitute an EV population. Thus, naturally, the present invention pertains both to individual EVs and populations comprising EVs, as will be clear to the skilled person. The dosages of EVs when applied *in vivo* may naturally vary considerably depending on the disease to be treated, the administration route, the therapeutic activity, effects, and potency of the NA cargo molecule, any targeting moieties present on the EVs, the pharmaceutical formulation, etc. Furthermore, the EVs of the present invention may also comprise additional therapeutic agents, in addition to the NA cargo molecule. In some embodiments, the additional therapeutic agent may be at least one therapeutic small molecule drug. In some embodiments, the therapeutic small molecule drug may be selected from the group consisting of DNA damaging agents, agents that inhibit DNA synthesis, microtubule and tubulin binding agents, anti-metabolites, inducers of oxidative damage, anti-angiogenics, endocrine therapies, anti-estrogens, immuno-modulators such as Toll-like receptor agonists or antagonists, histone deacetylase inhibitors, inhibitors of signal transduction such as inhibitors of kinases, inhibitors of heat shock proteins, retinoids, inhibitors of growth factor receptors, anti-mitotic compounds, anti-inflammatories, cell cycle regulators, transcription factor inhibitors, and apoptosis inducers, and any combination thereof. In further embodiments, the additional therapeutic agent may be an additional therapeutic NA-based agent. Such additional nucleic acid-based therapeutic agents may be selected from the group comprising single-stranded RNA or DNA, double-stranded RNA or DNA, oligonucleotides such as siRNA, splice-switching RNA, CRISPR guide strands, short hairpin RNA (shRNA), miRNA, cyclic dinucleotides, antisense oligonucleotides, polynucleotides such as mRNA, plasmids, or any other RNA or DNA vector. Of particular interest are nucleic acid-based agents which are chemically synthesized and/or which comprise chemically modified nucleotides such as 2'-O-Me, 2'-O-Allyl, 2'-O-MOE, 2'-F, 2'-CE, 2'-EA 2'-FANA, LNA, CLNA, ENA, PNA, phosphorothioates, tricyclo-DNA, etc. In yet further embodiments, the EVs as per the present invention may comprise additional therapeutic agents which may be proteins and/or peptides. Such proteins and/or peptides may be present inside of the EVs, inserted into the EV membrane or in association with the EV membrane, or may be protruding from the EV into the extravesicular environment. Such therapeutic protein and/or peptide agents may be selected from a group of non-limiting examples including: antibodies, intrabodies, single chain variable fragments (scFv), affibodies, bi- and multispecific antibodies or binders, affibodies, darpins, receptors, ligands, transporters, lysosomal proteins, enzymes, enzymes for e.g. enzyme replacement therapy or gene editing, tumor suppressors (non-limiting examples include p53, p21, pVHL, APC, CD95, ST5, YPEL3, ST7, and/or ST15) viral or bacterial inhibitors, cell component proteins, DNA and/or RNA binding proteins, DNA repair inhibitors, nucleases, proteinases, integrases, transcription factors, growth factors, apoptosis inhibitors and inducers, toxins (for instance pseudomonas exotoxins), structural proteins, neurotrophic factors such as NT3/4, brain-derived neurotrophic factor (BDNF) and nerve growth factor (NGF), ion channels, membrane transporters, proteostasis factors, proteins involved in cellular signaling, translation- and transcription related proteins, nucleotide binding proteins, protein binding proteins, lipid binding proteins, glycosaminoglycans (GAGs) and GAG-binding proteins, metabolic proteins, cellular stress regulating proteins, inflammation and immune system regulating proteins, mitochondrial proteins, and heat shock proteins, etc.

In a first aspect the present invention relates to EVs and compositions of EVs each of which comprise an NA cargo and a PolyA Binding Protein (PABP) as a stabilizing factor for the NA cargo, said NA cargo having a contiguous stretch of adenosine bases and/or a polyA tail to which the PABP binds. The terms "PolyA binding protein" or "PABP" shall be understood to include any protein capable of binding to a contiguous stretch of adenosine bases, i.e. a PolyA region (often referred to as a PolyA tail). The term PAPB includes: cytosolic poly-A binding proteins (such as PABPC1, PABPC3 and iPABP), nuclear polyA binding proteins such as PABP1, PABPN1, X-linked PAPB (PABPC5), and other proteins capable of binding to polyA regions such as LARP4A, KPAF4, DAZL, Coronavirus N capsid protein, PABP1 from *Trypanosome Brucei,* Human La proteins, Pan3L, as well as mutated or modified forms of PABP and fragments or domains of polyA binding proteins such as the N-Terminal domain (NTD), C-Terminal domain (CTD), PABP delta C-terminal or one or more of the RNA recognition motifs (RRMs) of polyA binding proteins. Other proteins with the ability to bind to contiguous stretches of adenosines such as catalytically inactive CAF1 or catalytically inactive CCR4-exonuclease are also included within the scope of the invention. These terms shall be considered to cover PABPs of any origin (prokaryotic or eukaryotic). Specific examples of PABPs from different species include: PABPC1 (Homo sapiens (Hs), Mus musculus (Mm), Xenopus laevis (XI)), PABPC2, PABPC3, PABP4 (hs, Mm, XI), PABP5 (Hs and Mm), EPAB (embryonic PABP, Hs, Mm, XI), tPABP (Hs and Mm), Dm PABP1 (Dm), PAB-1 (Ce) and PAB-2 (Ce), PAB-1 (Cb) and PAB-2 (Cb) PyPABP1 and PyPABP2 (*Plasmodium yoelii*)*,* PAB1 - PAB8 (*Arabidopsis*), LiPABP (*Leishmania infantum*), Nab2p and Pab1 (*S. cerevisiae*), Pab2 and Nab2 (*S. pombe*), mouse heterogeneous nuclear protein Q (hnRNP-Q2/SYNCRIP). The term "PolyA" is used interchangeably with "poly-adenylation" and shall be interpreted as any contiguous stretch of adenosine residues, either at the end of the NA cargo molecule or within the NA cargo molecule. The contiguous stretch of adenosine residues may be 10, 20, 50, 70, 100, 150, 200, 220, 250, 270 or 300 residues in length or more, or any other number in between. The present invention may be considered to encompass derivatives of these sequences which have at least a 50%, 60%, 70%, 75%, 80%, 85%, 90%, 95% or 99% homology to these sequences. The benefit of employing PAPB in the EVs and compositions of EVs of the present invention is that PABP binds to the polyA tail of the NA cargo and stabilizes the NA in producer cells thus increasing the stability and therefore the half-life of the NA in the producer cells resulting in higher levels of NA cargo loading either by passive uptake into EVs or by active uptake into EVs with the assistance of RNA binding fusion proteins (discussed below). Furthermore, PABP stabilizes and protects the NA cargo whilst the NA is in the EV, leading to an increased shelf life of product and when administered to a subject increasing half-life is key to potent therapeutic activity. Further still the presence of PABP stabilizes and protects the NA cargo from degradation once it is delivered into recipient cells. This longer half-life in the target/recipient cell therefore leads to more protein being translated from each delivered mRNA in the case when the cargo is mRNA. The speed of translation also increases because PABP assists in the formation of the translation initiation complex. By speeding up the formation of the translation initiation complex and facilitating the recruitment of small ribosomal subunit to the mRNA cargo PAPB allows the mRNA to be translated faster and in preference to other endogenous mRNAs. The formation of the translation initiation complex also permits cycling of the ribosome once it has attached to the mRNA due to the looped conformation of the mRNA which leads to faster protein production (more details below). The PABP therefore enables the mRNA to persist longer in the producer and recipient cells due to its increased stability and also to be translated faster and more frequently. The benefit of using mutated forms of PAPB is that the protein can be engineered to alter the binding affinity for the NA cargo, for instance to increase the binding affinity so as to result in even longer half-life of the cargo mRNA due to longer binding of the PAPB or to modify the PABP interaction with the translation initiation complex again to improve the affinity such that the complex forms faster and/or maintains its form longer so as to increase the length of time the mRNA is actively translated for, thus increasing the number of proteins generated from each mRNA cargo molecule. The benefit of using full, or nearly full length PAPB is that whilst the NTD interacts with the mRNA polyA tail at the 3' end of the mRNA cargo the CTD interacts with various translation initiation factors such as EIF4G (part of the EIF4F complex) at the 5' cap of the mRNA. This binding forms the characteristic loop structure of the mRNA needed for efficient recruitment of the ribosome and rapid recycling of terminating ribosomes from the 3' to the 5' end of enabling efficient cycles of translation from the same NA cargo before the mRNA decays or is degraded. Such ribosome recycling increases the number of protein products produced from each mRNA and thus improves the bioactive delivery and therapeutic efficacy of the cargo mRNA. The benefits of using only certain fragments or domains of PAPB is that these fragments/domains are smaller than full length PAPB and thus are easier to load into EVs compared to full length PABP due to their smaller size, either when PABP is overexpressed from a polynucleotide construct in the EV-producing cell or when PABP is fused to a fusion protein comprising an exosomal polypeptide and an NA-binding protein and/or domain (for enhanced EV loading of the NA cargo). It is possible to genetically engineer the PABP to include, for instance, only the NTD which would maintain its ability to stabilize the mRNA by binding to the polyA tail but would result in a smaller cargo of mRNA+PABP(NTD) which would be more easily loaded into EVs either by passive loading or by the assistance of the fusion proteins of the invention (discussed below). In a preferred embodiment the NA cargo is both stabilized by being bound by PABP and is actively loaded into the EVs by way of being bound by the fusion protein (typically comprising at least an exosomal protein fused to at least one NA-binding domain).

The present invention relates to EVs and compositions of EVs each of which comprise a translation initiation factor along with the NA cargo and a PolyA Binding Protein (PABP). The term translation initiation factor (TIF) shall be understood to relate to any protein factor that assists with the formation of the translation initiation complex or recruitment to, formation of, or maintenance of the ribosome at the translation initiation complex. Preferably the TIF is a eukaryotic TIF such as EIF4E, EIF4G, EIF1, EIF1A, EIF2, EIF3, EIF5, EIF5B and/or EIF6. As mentioned above the presence of the translation initiation complex is highly beneficial since it allows the mRNA cargo to form into a loop structure which facilitates ribosome recycling resulting in more protein product produced per mRNA molecule. This means that for every dose of EV the resulting therapeutic effect will be higher since more therapeutic protein is produced in the recipient cells. The present invention therefore relates in a specific embodiment to EVs which not only have mRNA cargo which is stabilized by the presence of PAPB (meaning an increased half-life of the mRNA in the EV-producer cell, in the EV and in the recipient cell) but the inclusion of a TIF, such as EIF4E, into the EV along with the stabilized mRNA cargo means that the stabilized cargo mRNA is delivered in a state which is primed and ready to be translated meaning that the more of the mRNA cargo will be delivered into the recipient cell in a highly bioactive form.

The terms "exosomal polypeptide", "exosomal protein", "exosomal carrier protein", "EV protein" and "EV polypeptide" and are used interchangeably herein and shall be understood to relate to any polypeptide that can be utilized to transport a polypeptide construct (which typically comprises, in addition to the exosomal polypeptide, an NA-binding domain, e.g. a polypeptide comprising an NA-binding domain) to a suitable vesicular structure, i.e. to a suitable EV. More specifically, these terms shall be understood as comprising any polypeptide that enables transporting, trafficking or shuttling of a fusion protein construct to a vesicular structure, such as an EV. Furthermore, these terms shall be understood to comprise any polypeptide that is naturally present and/or enriched in EVs (preferably exosomes) from any cell source. Examples of such exosomal polypeptides are for instance CD9, CD53, CD63, CD81, CD54, CD50, FLOT1, FLOT2, CD49d, CD71 (also known as the transferrin receptor) and its endosomal sorting domain, i.e. the transferrin receptor endosomal sorting domain, CD133 , CD138 (syndecan-1), CD235a, ALIX, AARDC1, Syntenin-1, Syntenin-2, Lamp2a, Lamp2b, syndecan-2, syndecan-3, syndecan-4, TSPAN8, TSPAN14, CD37, CD82, CD151, CD231, CD102, NOTCH1, NOTCH2, NOTCH3, NOTCH4, DLL1, DLL4, JAG1, JAG2, CD49d/ITGA4, ITGB5, ITGB6, ITGB7, CD11a, CD11b, CD11c, CD18/ITGB2, CD41, CD49b, CD49c, CD49e, CD51, CD61, CD104, CD2, CD3 epsilon, CD3 zeta, CD13, CD18, CD19, CD30, TSG101, CD34, CD36, CD40, CD40L, CD44, CD45, CD45RA, CD47, CD86, CD110, CD111, CD115, CD117, CD125, CD135, CD184, CD200, CD279, CD273, CD274, CD362, COL6A1, AGRN, EGFR, GAPDH, GLUR2, GLUR3, HLA-DM, HSPG2, L1CAM, LAMB1, LAMC1, LFA-1, LGALS3BP, Mac-1 alpha, Mac-1 beta, MFGE8, PTGFRN, SLIT2, STX3, TCRA, TCRB, TCRD, TCRG, VTI1A, VTI1B, Fibronectin, Rab7, 14-3-3 zeta/delta, 14-3-3 epsilon, HSC70, HSP90, HSPA13 other exosomal polypeptides, and any combinations or derivatives thereof, but numerous other polypeptides capable of transporting a polypeptide construct to an EV are comprised within the scope of the present invention. In some embodiments the exosomal protein is a transmembrane protein. In several embodiments of the present invention, at least one exosomal polypeptide is fused to NA-binding domain, in order to form a fusion protein present in an EV for aiding the loading of the NA cargo molecule. Such fusion proteins may also comprise various other components to optimize their function(s), including linkers, transmembrane domains, cytosolic domains, multimerization domains, domains for release of the NA-binding domain from the exosomal polypeptide, etc.

The terms "NA-binding domain" or "NA-binding polypeptide" or "NA-binding protein" are used interchangeably herein and relate to any protein or any domain of a protein that is capable of binding to a stretch of nucleotides, nucleosides and/or nucleobases. The NA-binding domains may bind to RNA, DNA, mixmers of RNA and DNA, particular types of NAs such as shRNA, miRNA, mRNA, gRNA, pri-miRNA, pre-miRNA, circular RNA, piRNA, tRNA, rRNA, snRNA, IncRNA, ribozymes, mini-circle DNA, plasmid DNA, etc. Furthermore, the NA-binding domain(s) may also bind to chemically modified nucleotides such as 2'-O-Me, 2'-O-Allyl, 2'-O-MOE, 2'-F, 2'-CE, 2'-EA 2'-FANA, LNA, CLNA, ENA, PNA, phosphorothioates, tricyclo-DNA, etc. Furthermore, the NA-binding domains may also bind to either particular sequences of NAs, to domains such as repeats, or to NA motifs, such as stem loops or hairpins. Such binding sites for the NA-binding domains may be naturally occurring in the NA cargo molecule of interest and/or may be engineered into the NA cargo molecule to further enhance EV loading and bioactive delivery. The binding affinity of the NA-binding domain for the nucleic acid is such that the nucleic acid is bound with high enough affinity to be shuttled into the exosomes but the affinity of binding is not so high as to prevent the subsequent release of the nucleic acid into the target cell such that the nucleic acid is bioactive once delivered to the target cell.

The present invention relates in some embodiments to NA-binding proteins, in particular RNA-binding and DNA-binding proteins and their NA-binding domains. Non-limiting examples of NA-binding proteins are hnRNPA1, hnRNPA2B1, DDX4, ADAD1, DAZL, ELAVL4, IGF2BP3, SAMD4A, TDP43, FUS, FMR1, FXR1, FXR2, EIF4A13, the MS2 coat protein, as well as any domains, parts or derivates, thereof. More broadly, particular subclasses of RNA-binding proteins and domains, e.g. mRNA binding proteins (mRBPs), pre-rRNA-binding proteins, tRNA-binding proteins, small nuclear or nucleolar RNA-binding proteins, non-coding RNA-binding proteins, miRNA-binding proteins, shRNA-binding proteins and transcription factors (TFs). Furthermore, various domains and derivatives may also be used as the NA-binding domain to transport an NA cargo into EVs. Non-limiting examples of RNA-binding domains include small RNA-binding domains (RBDs) (which can be both single-stranded and double-stranded RBDs (ssRBDs and dsRBDs) such as DEAD, KH, GTP_EFTU, dsrm, G-patch, IBN_N, SAP, TUDOR, RnaseA, MMR-HSR1, KOW, RnaseT, MIF4G, zf-RanBP, NTF2, PAZ, RBM1CTR, PAM2, Xpo1, Piwi, CSD, and Ribosomal_L7Ae. Such RNA-binding domains may be present in a plurality, alone or in combination with others, and may also form part of a larger RNA-binding protein construct as such, as long as their key function (i.e. the ability to transport an NA cargo of interest, e.g. an mRNA or a short RNA) is maintained.

In preferred embodiments the present invention relates to two groups of NA-binding domains, namely PUF proteins and CRISPR-associated polypeptides (Cas), specifically Cas6 and Cas13, as well as various types of NA-binding aptamers. The present invention uses the term PUF proteins to encompass all related proteins and domains of such proteins (which may also be termed PUM proteins), for instance human Pumilio homolog 1 (PUM1), PUMx2 or PUFx2 which are duplicates of PUM1, etc., or any NA-binding domains obtainable from any PUF (PUM) proteins. PUF proteins are typically characterized by the presence of eight consecutive PUF repeats, each of approximately 40 amino acids, often flanked by two related sequences, Csp1 and Csp2. Each repeat has a 'core consensus' containing aromatic and basic residues. The entire cluster of PUF repeats is required for RNA binding. Remarkably, this same region also interacts with protein co-regulators, and is sufficient to rescue, to a large extent, the defects of a PUF protein mutant, which makes the PUF proteins highly suitable for mutations used in the present invention. Furthermore, PUF proteins are highly preferred examples of releasable NA-binding domains which bind with suitable affinity to NA cargo molecules, thereby enabling a releasable, reversible attachment of the PUF protein to the NA cargo. PUF proteins are found in most eukaryotes and is involved in embryogenesis and development. PUFs has one domain that binds RNA that is composed of 8 repeats generally containing 36 amino acids, which is the domain typically utilized for RNA binding in this patent application. Each repeat binds a specific nucleotide and it is commonly the amino acid in position 12 and 16 that confer the specificity with a stacking interaction from amino acid 13. The naturally occurring PUFs can bind the nucleotides adenosine, uracil and guanosine, and engineered PUFs can also bind the nucleotide cytosine. Hence the system is modular and the 8-nucleotide sequence that the PUF domain binds to can be changed by switching the binding specificity of the repeat domains. Hence, the PUF proteins as per the present invention can be natural or engineered to bind anywhere in an RNA molecule, or alternatively one can choose PUF proteins with different binding affinities for different sequences and engineer the RNA molecule to contain said sequence. There is furthermore engineered and/or duplicated PUF domains that bind 16-nucleotides in a sequence-specific manner, which can also be utilized to increase the specificity for the NA cargo molecule further. Hence the PUF domain can be modified to bind any sequence, with different affinity and sequence length, which make the system highly modular and adaptable for any RNA cargo molecule as per the present invention. PUF proteins and regions and derivatives thereof that may be used as NA-binding domains as per the present invention include the following non-limiting list of PUF proteins: FBF, FBF/PUF-8/PUF-6,-7,-10, all from C. elegans; Pumilio from D. melanogaster; Puf5p/Mpt5p/Uth4p, Puf4p/Ygl014wp/Ygl023p, Puf5p/Mpt5p/Uth4p, Puf5p/Mpt5p/Uth4p, Puf3p, all from S. cerevisiae; PufA from Dictyostelium; human PUM1 (Pumilio 1, sometimes known also as PUF-8R) and any domains thereof, polypeptides comprising NA-binding domains from at least two PUM1, any truncated or modified or engineered PUF proteins, such as for instance PUF-6R, PUF-9R, PUF-10R, PUF-12R, and PUF-16R or derivatives thereof; and X-Puf1 from Xenopus. Particularly suitable NA-binding PUFs as per the present invention includes the following: PUF 531, PUF mRNA loc (sometimes termed PUFengineered or PUFeng), and/or PUFx2, (sequences of which are available in PCT/EP2018/080681) and any derivatives, domains, and/or regions thereof. The PUF/PUM proteins are highly advantageous as they may be selected to be of human origin.

In embodiments where PUF proteins CRISPR-associated polypeptides (Cas), specifically Cas6 and Cas13, and/or various types of NA-binding aptamers, are used the present invention advantageously leads to a releasable loading system which is absent in the prior art. Thus, importantly and in complete contrast to the prior art, the present invention relates to EVs loaded with NA cargo molecules with the aid of releasable NA-binding domains, wherein said NA-binding domains form part of fusion polypeptides with exosomal polypeptides. The NA-binding domains of the present invention have been selected to allow for programmable, modifiable affinity between the NA-binding domain and the NA cargo molecule, enabling production of EVs comprising fusion polypeptides comprising the NA-binding domain and at least one NA cargo molecule, wherein the NA-binding domain of the fusion polypeptide construct interacts in a programmable, reversible, modifiable fashion with the NA cargo molecule, allowing for both loading into EVs and release of the NA cargo molecule either in EVs and/or in or in connection with target cells.

Thus, in advantageous embodiments, the present invention relates to eukaryotic NA-binding proteins fused to exosomal proteins. In a preferred embodiment, the NA-binding domain(s) is(are) from the PUF family of proteins, for instance PUF531, PUFengineered, and/or PUFx2. Importantly, PUF proteins are preferably used in the EV-mediated delivery of mRNA due to the sequence-specificity of the PUF proteins which enables highly controlled and specific loading of the mRNA drug cargo. In preferred embodiments, the PUF protein(s) are advantageously combined with either transmembrane or soluble exosomal proteins. Advantageous fusion protein constructs include the following non-limiting examples: CD63-PUF531, CD63-PUFx2, CD63-PUFengineered, CD81-PUF531, CD81-PUFx2, CD81-PUFengineered, CD9-PUF531, CD9-PUx2, CD9-PUFengineered, and other transmembranebased fusion proteins, preferably based on tetraspanin exosomal proteins fused to one, two or more PUF proteins. Advantageous fusion proteins comprising PUF proteins and at least one soluble exosomal protein include the following non-limiting examples: syntenin-PUF531, syntenin-PUx2, syntenin-PUFengineered, syndecan-PUF531, syndecan-PUx2, syndecan-PUFengineered, Alix-PUF531, Alix-PUx2, Alix-PUFengineered, as well as any other soluble exosome protein fused to a PUF protein.

The fact that the PUF proteins have modifiable sequence-specificity for the target NA cargo molecule makes them ideal NA-binding domains for fusing to exosomal polypeptide partner(s). Thus, in preferred embodiments of the present invention, the EVs are loaded with NA cargo molecules using releasable NA-binding domains (as part of fusion proteins with exosomal proteins), wherein the interaction between the NA-binding domain and the NA cargo molecule is advantageously based on specificity for a target nucleotide sequence and not based on a target nucleotide secondary structure (as secondary structures do not enable sequence specificity). In preferred embodiments, the NA cargo molecule is engineered to comprise and/or naturally comprises the target nucleotide sequence for the PUF protein chosen as the NA-binding domain. Such target nucleotide sequences may as abovementioned, for example, be part of the 3'UTR of an mRNA or may be introduced into any NA cargo molecule such as an mRNA, shRNA, miRNA, IncRNA, DNA etc., allowing for the PUF protein to bind to the NA cargo molecule. The PUF binding site on the NA cargo molecule is typically longer than the sequence bound by many other RNA-binding proteins, such as MS2 which merely recognizes 4 nucleotides and a stem loop in combination, so the preferred stretch of nucleotides on the target binding site may be for instance 5 nucleotides (nt), 6 nt, 7 nt, 8 nt, 9 nt, 10 nt, 11 nt, 12 nt, 13 nt, 14 nt, 15 nt, 16 nt, 17 nt, 18 nt, 19 nt, or even 20 nt and longer, depending on the need for modifiable sequence specificity of the NA-binding domain. In a preferred embodiment, the PUF protein is specific for a natural and/or artificially occurring NA cargo molecule binding site which is 6 nt, 8 nt, 9 nt, 10 nt, 12 nt, or 16 nt in length.

CRISPR-associated polypeptides (Cas) represent another group of NA-binding domains, and may include in particular Cas6 and Cas13 as well as any other RNA binding Cas molecule. Cas6 binds precursor CRISPR RNA (crRNA) with high affinity and processes it for later incorporation into for example Cas9. The cleavage rate of the RNA molecule can be modulated and highly defined, hence the association time between the RNA molecule and Cas6 can also be defined in a very accurate fashion, which is important for the purposes of the present invention. Mutant versions of Cas6 or Cas13 may be used which have been mutated to increase or decrease efficiency of RNA cleavage. Mutant versions of Cas6 or Cas13 may be used which have been mutated to increase or decrease the affinity of RNA binding. This will be an advantage for instance when the RNA cargo molecule is to be released in the recipient cell. The defined association time can then be modulated to release the RNA molecule inside the vesicles, but not in the producer cell. The RNA sequence that Cas6 can recognize can be engineered to be inserted into an NA molecule of interest. Cas13 can be engineered to only bind its defined RNA target and not degrade it. By changing the sequence of the sgRNA molecule the Cas13-sgRNA complex can be modulated to bind any RNA sequence between 20-30 nucleotides. For instance, the use of NA-binding domains from Cas proteins is especially advantageous for the delivery of short RNAs, for instance shRNAs or miRNAs. In such instances the cleavage activity of the selected Cas polypeptides may be used to release e.g. an shRNA cargo molecule from a binding site to which e.g. Cas6 has bound. Furthermore, as is the case with the PUF proteins, Cas proteins are highly preferred examples of releasable NA-binding domains which bind with suitable affinity to NA cargo molecules, thereby enabling a releasable, reversible attachment of the Cas protein to the NA cargo. As with the PUF-based NA-binding domains, the Cas proteins represent a releasable, irreversible NA-binding domain with programmable, modifiable sequence specificity for the target NA cargo molecule, enabling higher specificity at a lower total affinity, thereby allowing for both loading of the NA cargo into EVs and release of the NA cargo in a target location.

Thus, combining PUF- or Cas-mediated loading into EVs of NA cargo molecules, such as mRNA, shRNA and miRNA, with PABP-mediated stabilization of the NA cargo molecules (via interaction between PABP and a stretch of adenosine residues of the NA cargo molecule) represents a highly advantageous embodiment of the present invention, resulting in efficient NA cargo delivery and bioactivity. As abovementioned, in all embodiments of the present invention PABP may be translated separately from the same or from a different polynucleotide construct, or it may be included into a fusion polypeptide for EV loading. Polypeptide constructs for loading of NA cargo into EVs may be described schematically as follows (the below notation is not to be construed as illustrating a C and/or N terminal direction or any particular sequence of the components *per se,* it is merely meant to illustrate suitable components of the fusion polypeptide):
Exosomal polypeptide - NA binding domain - PABP

In certain embodiments, the fusion polypeptide further comprises a transcription initiation factor (for instance EIF), as per the schematic illustration below:
Exosomal polypeptide - NA binding domain - PABP - EIF

Furthermore, as mentioned herein, polypeptide construct such as the schematic illustrations above may also comprise linkers, domains for multimerization, cleavage sites for protease cleavage or for spontaneous release, etc., depending on the desired functionality of the fusion polypeptide.

NA aptamer-binding domains are another group of NA-binding domains as per the present invention. Such NA aptamer-binding domains are domains, regions, stretches of amino acids, or entire polypeptides or proteins that can be bound with specificity by NA-based aptamers. Aptamers are RNA sequences that form secondary and/or tertiary structures to recognize molecules, similar to the affinity of an antibody for its target antigen. Hence these RNA molecules can recognize specific amino acid sequences with high affinity. RNA aptamers are applied in the present invention by inserting particular nucleotide sequences into the NA molecule to recognize specific amino acid sequences. Such amino acid sequences can be engineered into and/or next to the exosomal carrier polypeptide to enable the aptamer (which is engineered into and/or next to the NA cargo molecule) to bind to it, thereby shuttling the NA cargo molecule into EVs with the aid of the exosomal polypeptide. Two aptamers with suitable characteristics are a His-aptamer with high affinity for a stretch of histidine (His) amino acids and an aptamer towards the HIV Tat domain. The aptamer sequence(s) are preferably inserted in the 3' and/or 5' untranslated region of an mRNA. Two or more aptamers can also be combined into one mRNA cargo molecule to increase the specificity and avidity to the exosomal carrier protein. Importantly, all the NA-binding domains of the present invention provide for programmable, sequence-specific, reversible, releasable binding to the NA cargo molecule, which is in complete contrast to the high-affinity, irreversible binding to RNA found in the prior art. In preferred embodiments of the present invention, the NA-binding domains are either PUF proteins or Cas proteins, due to their easily programmable nature and sequence specificity combined with their reversible, releasable binding to NA cargo molecules. Importantly, the sequence specificity of Cas proteins and PUF proteins as NA-binding domains is preferably based on interaction with at least 6 nt, preferably at least 8 nt on the target NA molecule, which when combined with a low-affinity interaction allows for high productive EV-mediated delivery of the NA cargo molecule. The at least 6 nt binding site on the NA cargo molecule is preferably present in a contiguous sequence of nucleotides. The binding site of the NA cargo molecule thus preferably corresponds in length to two codons. Aptamermediated loading is advantageously combined with PABP-mediated stabilization enhancement, especially as NA-binding aptamers are particularly short, meaning that the fusion polypeptide construct (which may comprise PABP itself) will be relatively condensed in size.

Thus, in another important aspect the present invention relates to extracellular vesicles (EVs) which comprise PABP as well as the NA cargo molecule in combination with at least one fusion polypeptide comprising at least one nucleic acid (NA)-binding domain and at least one exosomal polypeptide. As a result of presence of the NA-binding domain fused to at least one EV polypeptide, the EVs typically comprise a higher copy number of the NA cargo molecule. Using this strategy the number of intact, stable NA cargo molecules that are comprised in each and every EV is considerable, which is a clear improvement over the prior art which normally achieves a very low loading efficacy and often exhibits problems associated with mRNA stability. In the case of the present invention, the inventive design of the fusion polypeptide constructs means that the at least one NA cargo molecule is very efficiently transported into the EV (with the help of the fusion polypeptide) followed by a significantly improved release process, with both processes being enhanced by the increased stability mediated by the presence of PABP. The releasable nature of the binding between the NA-binding domain (which is comprised in the fusion polypeptide) and the NA cargo molecule is an important aspect of the present invention, as it allows for binding of NA cargo molecules in the EV-producing cells (where NA cargo molecules are normally overexpressed) while enabling delivery of bioactive NA molecules in and/or near the target cell.

As mentioned above, in embodiments employing PUF proteins and CRISPR-associated polypeptides (Cas), specifically Cas6 and Cas13, and/or various types of NA-binding aptamers the invention additionally has the advantageous effect of achieving a programmable, lower affinity interaction between the NA-binding domain and the NA cargo molecules enables the present invention to efficiently load EVs in EV-producing cells, whilst also enabling release of NA cargo in suitable locations (typically inside a target cell) where the lower affinity and the releasable nature of the interaction between the NA cargo molecule and the NA-binding domain is highly advantageous. Furthermore, unlike the prior art which discloses MS2 as a high-affinity RNA-binding protein binding to 4 nts and a stem loop, the present invention allows for sequence-specific low-affinity or medium-affinity binding to stretches of nucleotides that are longer and thereby more specific, for instance 6 nt in length, or 8 nt in length.

The longer length of binding site enables a range of different mutations to be introduced which generate binding sites with a range of modified binding affinities, thus producing the programmable lower affinity interactions mentioned above. For instance, introduction of a single point mutation into a 6 or 8 nucleotide region will subtly modify the binding affinity, whereas, even a single mutation in the shorter 4 nucleotide binding region of MS2 is known to significantly affect the binding affinity of MS2 for the RNA. The longer length of nucleic acid provides more scope to introduce one or more mutations which affect the binding affinity of the protein for the nucleic acid. Similarly, requiring a longer stretch of nucleotides to be bound results in a larger number of amino acids which are capable of interacting with the longer nucleotide sequence and thus providing more possibilities for mutating those interacting amino acids and again producing a larger range of possible protein mutants with a variety of binding affinities. Both the longer nucleotide binding site and the larger protein binding sites of PUF, Cas6 and Cas 13 provide advantages in enabling a greater range of affinities to be achieved by mutation than could be achieved by mutation of the MS2 protein or the MS2 RNA sequence. Thus, this longer sequence affords greater possibilities to engineer the nucleic acid and/or the binding protein to tailor the binding affinity specifically to an individual cargo of interest if needed to improve the release of that cargo nucleic acid. As has been discussed above, the ability to control the affinity of binding to the nucleotide cargo and thus modify and control the releasability of the nucleotide cargo is a significant advantage of the present invention over the prior art resulting in delivery and release of bioactive nucleic acids. MS2 can, of course, be used advantageously in situations when a high affinity binding is required.

In one embodiment, the NA cargo molecule may be selected from the group comprising shRNA, miRNA, mRNA, gRNA, pri-miRNA, circular RNA, piRNA, tRNA, rRNA, naRNA, IncRNA, ribozymes, mini-circle DNA, plasmid DNA, cyclic dinucleotides, but essentially any type of NA molecule can be comprised in the EVs as per the present invention. Both single and double stranded NA molecules are within the scope of the present invention, and the NA molecule may be naturally occurring or may be a chemically synthesized molecule which may comprise chemically modified nucleotides such as 2'-O-Me, 2'-O-Allyl, 2'-O-MOE, 2'-F, 2'-CE, 2'-EA 2'-FANA, LNA, CLNA, ENA, PNA, phosphorothioates, tricyclo-DNA, etc. Importantly, although the present invention is highly suitable for endogenous loading of NA cargo molecules (for instance mRNA, circular RNA, shRNA etc.) it is also applicable to loading with exogenous NA molecules which may be loaded by exposing EV-producing cells to the NA molecule in question and/or by co-incubation, electroporation or formulating with the EVs per se.

In a specific embodiment the EV of the present invention comprises PABP to stabilize the NA cargo and the NA cargo is actively loaded into the EV with the assistance of a fusion protein comprising an exosomal protein fused to a NA-binding domain, preferably the fusion protein comprises exosomal protein CD63 or a portion thereof fused to the nucleic acid binding protein PUFeng. In a preferred embodiment, a polynucleotide construct as per the present invention comprises a coding region encoding for the fusion protein (comprising an exosomal protein, such as CD63 or syntenin or any other suitable EV protein, and an NA binding protein, such as PUFeng) and PABP or a fragment or domain thereof incorporated into the same polynucleotide construct either under the control of different promoters or separated by a P2A cleavage site which results in the separation of the two protein products at the translational level. The advantage of this embodiment is that the generation of stable cells to produce the EVs is simplified by requiring only the transfection of a single construct for the protein component of the engineered cell (the NA cargo may also in some embodiments be incorporated into the same polynucleotide construct, but it may also be present in a second construct, thereby creating a double stable engineered EV-producing cell).

In another embodiment the present invention comprises EVs comprising a fusion protein comprising an exosomal protein, an NA binding domain, and PABP or a fragment or domain thereof. This embodiment has the same benefit that the cells producing such a protein construct need only be transfected with a single protein-encoding construct resulting in a simpler generation of stable cells to produce said EV. Additionally, this arrangement has the benefit that the over expressed PABP is actively loaded into the EVs (because it is incorporated into the fusion protein) as a result of the exosomal protein component of the fusion protein and importantly that the PABP is located proximally to the NA cargo which has been dragged into the EV by the NA binding protein.

In another embodiment the PABP protein may be fused to an NA binding protein, the corresponding NA binding site would then be engineered into the NA cargo molecule so that the PABP overexpression is specifically targeted to the NA cargo rather than any other NA (for instance an mRNA present in the cytosol) which may be present naturally in the producer cells. This approach therefore results in increased stability of the target cargo NA.

In a specific embodiment the NA-binding domain is Cas6 or Cas13 and the NA cargo molecule is an shRNA or a miRNA. The combination of Cas6/Cas13 with shRNA cargo is advantageous because the innate activity of Cas6/Cas13 will result in the cleavage of the shRNA from the NA-binding domain(s) to which Cas6 or Cas13 have bound, thereby releasing the shRNA from the complex between the fusion protein and the NA-binding domain. Additionally, in order to enhance the stability of the shRNA, a stretch of adenosine resides may be engineered onto the shRNA, to enable binding and stabilization by PABP, but in the EV-producer cells and in the EVs per se. Once delivered to a target environment, it is surmised that Cas6 or Cas13 would then cleave off the therapeutically active shRNA part of the shRNA - NA binding site - polyA stretch polynucleotide, thereby releasing the shRNA and enabling it to carry out its gene silencing function.

In preferred embodiments of the present invention, the NA cargo molecules as per the present invention comprise (i) at least one binding site for the NA-binding domain of the fusion polypeptide and (ii) a therapeutic polynucleotide domain, i.e. the part of the NA cargo molecule which is responsible for exerting a biological effect, for instance the coding sequence of an mRNA or a silencing sequence of an shRNA or an miRNA. In preferred embodiments, the NA cargo molecule comprises at least two binding sites and even more preferably a higher number of binding sites, e.g. 3, 4, 5, 6, 7, 8, 9, 10, 15, or an even greater number. The inventors have realized that including 4-8 binding sites yields optimal loading of the NA cargo molecule into EVs without negatively impacting the release and bioactive delivery of the cargo. The binding sites for the NA-binding domain, can be genetically engineered into and/or flanking the 3' and/or 5' regions and/or by sequence optimization be placed in the coding region of the NA cargo molecule.

The NA cargo molecules as per the present invention may advantageously comprise at least one cleavage site between the at least one binding site and the therapeutic and or prophylactic polynucleotide domain, in order to enable release of the part of the NA cargo molecule that is responsible for its biological prophylactic and/or therapeutic activity.

Generally, the NA cargo molecule is intended to carry out a range of functions, for instance encode for a protein of interest (such as a protein with a therapeutic and/or prophylactic activity), silence a target nucleotide sequence via antisense interaction with the target, switch and/or block splicing, mediate cleavage of a target nucleotide sequence e.g. via RNase H-mediated cleavage or RISC complex mediated RNA interference (RNAi). In embodiments of particular interest, the NA cargo molecule may carry out more than one function, for instance it may encode for a protein of interest and comprise an NA sequence which may have e.g. a guiding function. A particularly advantageous example of this is an NA molecule encoding for a CRISPR-associated protein (such as Cas, Cas9 (in a non-limiting example Cas9 with accession number Q99ZW2), and/or Cas6) and also comprising a guide strand for directing the CRISPR-associated protein to a target sequence for gene editing and/or a correcting DNA strand for e.g. homology directed repair. In such embodiments, it is particularly advantages to include cleavage sites in the NA molecule, to enable release of either one or both of the protein encoded by the NA cargo molecule and the guide strand comprised in the NA cargo molecule.

Non-limiting examples of proteins of interest (Pols) that may be encoded for by the NA (in this embodiment advantageously an mRNA) cargo molecule include the following:: antibodies, intrabodies, single chain variable fragments (scFv), affibodies, bi- and multispecific antibodies or binders, receptors, ligands, enzymes for e.g. enzyme replacement therapy or gene editing, tumor suppressors, viral or bacterial inhibitors, cell component proteins, DNA and/or RNA binding proteins, DNA repair inhibitors, nucleases, proteinases, integrases, transcription factors, growth factors, apoptosis inhibitors and inducers, toxins (for instance pseudomonas exotoxins), structural proteins, neurotrophic factors such as NT3/4, brain-derived neurotrophic factor (BDNF) and nerve growth factor (NGF) and its individual subunits such as the 2.5S beta subunit, ion channels, membrane transporters, proteostasis factors, proteins involved in cellular signaling, translation- and transcription related proteins, nucleotide binding proteins, protein binding proteins, lipid binding proteins, glycosaminoglycans (GAGs) and GAG-binding proteins, metabolic proteins, cellular stress regulating proteins, inflammation and immune system regulating proteins, mitochondrial proteins, and heat shock proteins, etc. In one preferred embodiment, the encoded protein is a CRISPR-associated (Cas) polypeptide with intact nuclease activity which is associated with (i.e. carries with it) an RNA strand that enables the Cas polypeptide to carry out its nuclease activity in a target cell once delivered by the EV. Alternatively, in another preferred embodiment, the Cas polypeptide may be catalytically inactive, to enable targeted genetic engineering. Yet another alternative may be any other type of CRISPR effector such as the single RNA guided endonuclease Cpf1. The inclusion of Cpf1 is a particular preferred embodiment of the present invention, as it cleaves target DNA via a staggered double-stranded break, Cpf1 may be obtained from species such as, Acidaminococcus or Lachnospiraceae. In yet another exemplary embodiment, the Cas polypeptide may also be fused to a transcriptional activator (such as the P3330 core protein), to specifically induce gene expression. Additional preferred embodiments include proteins selected from the group comprising enzymes for lysosomal storage disorders, for instance glucocerebrosidases such as imiglucerase, alpha-galactosidase, alpha-L-iduronidase, iduronate-2-sulfatase and idursulfase, arylsulfatase, galsulfase, acid-alpha glucosidase, sphingomyelinase, galactocerebrosidase, galactosylceramidase, ceramidase, alpha-N-acetylgalactosaminidase, beta-galactosidase, lysosomal acid lipase, acid sphingomyelinase, NPC1, NPC2, heparan sulfamidase, N-acetylglucosaminidase, heparan-α-glucosaminide-N-acetyltransferase, N-acetylglucosamine 6-sulfatase, galactose-6-sulfate sulfatase, galactose-6-sulfate sulfatase, hyaluronidase, alphaN -acetyl neuraminidase, GlcNAc phosphotransferase, mucolipin1, palmitoylprotein thioesterase, tripeptidyl peptidase I, palmitoyl-protein thioesterase 1, tripeptidyl peptidase 1, battenin, linclin, alpha-D-mannosidase, beta-mannosidase, aspartylglucosaminidase, alpha-L-fucosidase, cystinosin, cathepsin K, sialin, LAMP2, and hexoaminidase. In other preferred embodiments, the Pol may be e.g. an intracellular protein that modifies inflammatory responses, for instance epigenetic proteins such as methylases and bromodomains, or an intracellular protein that modifies muscle function, e.g. transcription factors such as MyoD or Myf5, proteins regulating muscle contractility e.g. myosin, actin, calcium/binding proteins such as troponin, or structural proteins such as dystrophin, utrophin, titin, nebulin, dystrophin-associated proteins such as dystrobrevin, syntrophin, syncoilin, desmin, sarcoglycan, dystroglycan, sarcospan, agrin, and/or fukutin. The Pols are typically proteins or peptides of human origin unless indicated otherwise by their name, any other nomenclature, or as known to a person skilled in the art, and they can be found in various publicly available databases such as Uniprot, RCSB, etc.

The designs of the NA cargo molecule, the design of the construct encoding the PABP protein or the fragment thereof as well as the design of the fusion polypeptide constructs and the design of the construct encoding the translation initiation factor are key to providing stability to the NA cargo, as well as improving loading, release, bioactive delivery and efficient translation of the NA cargo, e.g. into target cells and/or into particular organs, tissues, and bodily compartments. The inventors have discovered that particularly advantageous embodiments are EVs comprising fusion polypeptides which comprises at least one exosomal polypeptide flanked on one or both sides by at least one NA-binding domain (i.e. at least one NA-binding domain either on one side or on each side). Alternatively, the NA-binding domain may in various instances by inserted into the exosomal polypeptide in at least one location (for instance on an extravesicular loop of e.g. CD63), for instance when it is desirable to display the NA-binding domain on the outside of the EV to enhance exogenous loading. The exosomal polypeptide may be flanked immediately C and/or N terminally, but the most advantageous design is to include a linker peptide between the exosomal polypeptide and the NA-binding domains, to provide spacing and flexibility for maintained activity of both the exosomal polypeptide(s) and the NA-binding domain(s). Such linkers may advantageously be glycineserine (GS) linkers containing a particular number of repeats. The inventors have realized that either 1 to 4 repeats are the most advantageous, providing enough flexibility without rendering the fusion polypeptide too unstructured, however longer linkers and non-GS-based linkers are also within the scope of the present application. Furthermore, as described above, PABP may in advantageous embodiments be fused to the fusion polypeptide comprising the NA-binding domain and the exosomal polypeptide. Linkers, release domains and cleavage sites as described above can advantageously be employed also in this type of fusion protein construct, to enable the different functional domains to carry out their respective activity (i.e. the EV protein trafficking the fusion protein into an EV; the NA-binding domain binding to an NA cargo molecule of interest; and, PABP binding to a stretch of adenosine residues to stabilize the NA cargo and enhance its biological activity). As above-mentioned, for applications involving exogenous loading of NA cargo molecules, EVs preferably comprise fusion polypeptides which comprises at least one exosomal polypeptide fused to at least one NA binding domain on its N terminal, and/or its C terminal and/or in any extravesicular (i.e. present outside of the EV) regions of the exosomal polypeptide, in order to expose the NA binding domain on the surface of exosome.

Design and selection of the exosomal polypeptide component of the fusion polypeptide construct is key to enable efficient EV formation, NA loading into the EVs, and also release of the NA cargo molecule.

As above-mentioned, the EVs as per the present are loaded with the NA cargo molecule with the aid of the fusion polypeptide. Without wishing to be bound by any theory, it is surmised that the loading takes place in connection with the formation of the EV inside the EV-producing cell or exogenously by incubating NA cargo molecule(s) with engineered EVs. The fusion polypeptide may normally bind to the NA cargo molecule whilst it is co-expressed in the EV-producing cell and transport it into the vesicle which is then released as an EV. As mentioned, the NA cargo molecule may be expressed in the same EV-producing cell as the fusion polypeptide and/or it may be loaded exogenously into an EV once the EV is formed and optionally purified. Co-expression in the EV-producing cell of the NA cargo is a highly advantageous embodiment, as the EV production takes place in a single step in a single cell, which enables scaling the process and simplifies both upstream and downstream processing. The NA cargo molecule (e.g. an mRNA, an shRNA, a miRNA, a circular RNA, a DNA, an antisense oligonucleotide, etc.) may be expressed from the same polynucleotide constructs as the fusion polypeptide construct which may optionally also comprise PABP, or each of the NA cargo molecule, the fusion polypeptide construct, and PABP may be expressed from separate constructs or in any combination. Each method has its advantages: the use of one construct ensures that the NA cargo molecule and the PABP and optionally also the fusion protein and/or the translation initiation factor (TIF) is translated/transcribed together whereas the use of more than one construct enables differential expression of the these components, e.g. a higher expression level of the PABP construct, the TIF construct, the fusion polypeptide and/or the NA cargo molecule. In preferred embodiments, the polynucleotide construct(s) from which the PABP and the NA cargo (and in certain embodiments the optional fusion polypeptide and/or the TIF) are expressed is advantageously stably introduced into the EV-producing cells, to enable consistent, reproducible and high-yield production of the NA-loaded EVs. In a preferred embodiment, the EV-producing cells are stably transfected and/or transduced with bicistronic or multicistronic vectors (also known as polynucleotide constructs or polynucleotides, etc.) comprising PABP and the NA cargo molecule (and in certain embodiments also the optional fusion protein and/or the TIF). Such bicistronic or multicistronic construct may comprise e.g. IRES element(s) or 2A peptide linkages, allowing for the expression of (i) the PABP, (ii) the NA cargo of interest, (ii) the fusion polypeptide comprising the NA-binding domain and the exosomal protein, and (iv) the TIF. In addition to using bicistronic or multicistronic vectors, multiple or bidirectional promoters represent another tractable method for stably inserting a single construct encoding for the two components of interest that are to be loaded into the EVs according to the present invention. Clearly, in alternative embodiments, two or more polynucleotide constructs (for instance plasmids) may also be transfected and/or transduced into EV-producing cells, although the use of single constructs may be advantageous as it may enable equimolar concentrations of the PABP and the NA cargo molecule *per se* (and optionally the fusion protein and/or the TIF) and as it may also simplify creating stable clonal cell lines. Importantly, the EV-producing cells are normally designed and engineered to overexpress the at least one polynucleotide construct, which allows for appropriate production of the NA cargo molecule at a suitable concentration in the EV-producing cell, thereby allowing for the reversible, releasable attachment of the NA-binding domain to the NA molecule. Overexpression of the polynucleotide(s) is an important tool that allows for creating a relatively high NA cargo molecule concentration in the EV-producing cell, while allowing at the same time for release of the NA cargo molecule in the target cell where the concentration of the NA cargo molecule is lower.

In a further embodiment, the EVs as per the present invention may comprise at least one targeting moiety, to enable targeted delivery to a cell, tissue, organ, organelle, and/or compartment of interest. Organs, tissues and cell types that may be targeted include: the brain, neuronal cells, the blood brain barrier, muscle tissue, the eye, lungs, liver, kidneys, heart, stomach, intestines, pancreas, red blood cells, white blood cells including B cells and T cells, lymph nodes, bone marrow, spleen and cancer cells. The targeting moiety may be comprised in the fusion polypeptide itself, which is especially advantageous when using an exosomal polypeptide with a transmembrane domain to enable display of the targeting moiety on the surface of the EVs. Targeting moieties may be proteins, peptides, single chain fragments or any other derivatives of antibodies, etc. The targeting moiety may also form part of a separate polypeptide construct which is comprised in the EV. Further, the fusion polypeptides comprised in the EVs of the present invention may also comprise various additional moieties to enhance the bioactive delivery. Such moieties and/or domains may include the following non-limiting examples of functional domains: (i) multimerization domains which dimerize, trimerize, or multimerize the fusion polypeptides to improve EV formation and/or loading, (ii) linkers, as above-mentioned, to avoid steric hindrance and provide flexibility, (iii) release domains, such as cis-cleaving elements like inteins, which have selfcleaving activity which is useful for release of particular parts of the fusion polypeptide and/or the NA cargo, (iv) RNA cleaving domains for improved release of the NA in recipient cells, for instance domains encoding for nucleases such as Cas6, Cas13, (v) endosomal escape domains, such as HA2, VSVG, GALA, B18, etc., and/or (vi) nuclear localization signals (NLSs).

The present invention also relates to various inventive modifications of the NA cargo molecule, which are key to ensure high efficiency of loading, release and bioactive delivery. For instance, by designing the NA cargo molecule to be either linear or circular one can increase or decrease aspects such as loading efficiency and stability. Furthermore, by optimizing the design of the sequence it is also possible to influence secondary and tertiary structures of the NA cargo, which can further facilitate loading, by facilitating the easy accessibility of NA binding domain to the target NA.

In yet another advantageous embodiment, the NA cargo molecule may comprise additional moieties to increase potency, either by enhancing loading, improving release, increasing tissue-specific activity, and/or increase the stability of the NA cargo molecule. For instance, the NA cargo molecule may comprise one or more of the following: (i) a site for miRNA binding, wherein such site optionally is tissue and/or cell type specific, to drive preferential cell and/or tissue specific activity, (ii) more than one PolyA tail (for instance 2 or 3 or even 4 PolyA tails), (iii) at least one stem loop structure in the 5' and/or 3' region, in order to inhibit nuclease degradation, (iv) an RNA polymerase to drive transcription of the NA cargo molecule in the embodiments when this is required, e.g. if the NA is DNA, (v) codon-optimized sequences to increase NA stability, in particular mRNA stability, (vi) at least one hybrid UTR in the 5' and/or 3' end to increase mRNA translation efficiency, and/or (vii) ribozyme(s). The polyA 'tail' or contiguous stretch of adenosine residues may be present at the end of the NA cargo molecule or positioned within other elements of the NA cargo molecule so long as the PAPB is still able to bind to the contiguous stretch of adenosines and thereby prevent or decrease degradation of the NA cargo.

Importantly the prior art makes no attempt to improve the translation of the mRNA once it is delivered to the recipient cells. The presence of the PABP and optionally also the TIF of the present invention recruits the translation initiation complex and the ribosome to enable rapid, repeated translation of the already stabilized mRNA cargo so as to increase the number of proteins translated from each cargo mRNA and hence increase the therapeutic effect of the loaded EV.

As above-mentioned, EVs are typically present not as single vesicles but in a substantial plurality of vesicles, and the present invention hence also relates to populations of EVs. In advantageous embodiments, the average number of NA cargo molecules per EV throughout such a population is above on average one (1) NA cargo molecule per EV, preferably above 10 NA cargo molecules per EV, and even more preferably above 100 or more NA cargo molecules per EV. However, throughout the population there may also be EVs which do not comprise any NA cargo molecules, and the present invention may thus also relate to populations of EVs which comprise on average less than one (1) NA cargo molecule per EV.

Importantly, the prior art typically merely yields loading of the NA cargo into a small fraction of the EVs. For instance, the TAMEL system described in US14/502,494 does not appear to enable quantifiable loading into EVs and specifically not into exosomes. This likely indicates that the TAMEL system results in zero to sub-single percentage loading of single EVs. The inventors of the TAMEL system reports that loading of an mRNA molecule into exosomes is enhanced at most 7-fold, whereas the present invention improves productive loading of e.g. mRNA and other NA cargo molecules by typically at least 10-fold, preferably at least 25-fold, but frequently by at least 50-fold, and preferably by at least 70-fold, most preferably by 100 fold or more, as compared to (i) EVs without NA-binding domain present in the fusion protein and/or without binding site for the NA-binding domain in the NA cargo molecule, (ii) EVs without the fusion protein *per se,* (iii) un-engineered EVs which are only passively loaded with the NA cargo molecule, and/or (iv) a given internal NA control molecule. Thus, the present invention provides for a way of loading considerably more stable and active NA cargo molecules into a given population of EVs, and importantly the present invention also enables loading a significantly higher proportion of EVs as compared to the prior art and that cargo NA is stabilized by the PABP and optionally also the TIF such that the half-life of the NA is increased allowing for more bioactive delivery of the NA which in the case of mRNA furthermore results in the mRNA being translated more efficiently and more frequently before the mRNA is degraded. In one embodiment, the present invention relates to EV populations wherein at least 5%, at least 10%, at least 20%, at least 50%, at least 70%, at least 75%, at least 80%, at least 85%, at least 90%, and/or at least 95% of all EVs comprise at least one copy of the NA cargo molecule in question. The prior art makes no attempt to improve the stability and thus half-life of the NA cargo and in fact makes no reference to this problem at all. Furthermore the prior art makes no attempt to improve the efficiency of translation of the mRNA once delivered to the recipient cell and again is silent with regard to this problem.

In advantageous embodiments, the fusion polypeptides may optionally further comprise additional regions, domains, sequences, and/or moieties endowing the polypeptide with particular functions. Non-limiting examples of additional domains comprised in the fusion polypeptide include (i) multimerization domains, (ii) linkers, (iii) release domains, (iv) RNA cleaving domains such as P2A, (v) endosomal escape moieties, (vi) protease specific cleavage sites, (vii) inteins and/or (viii) targeting moieties.

Multimerization domains enable dimerization, trimerization, or any higher order of multimerization of the fusion polypeptides, which increases the sorting and trafficking of the fusion polypeptides into EVs and may also contribute to increase the yield of vesicles produced by EV-producing cells. Linkers are useful in providing increased flexibility to the fusion polypeptide constructs, and also to the corresponding polynucleotide constructs, and may also be used to ensure avoidance of steric hindrance and maintained functionality of the fusion polypeptides. Release domains may be included in the fusion polypeptide constructs in order to enable release of particular parts or domains from the original fusion polypeptide. This is particularly advantageous when the release of parts of the fusion polypeptide would increase bioactive delivery of the NA cargo and/or when a particular function of the fusion polypeptide works better when part of a smaller construct. Suitable release domains may be cis-cleaving sequences such as inteins, light induced monomeric or dimeric release domains such as Kaede, KikGR, EosFP, tdEosFP, mEos2, PSmOrange, the GFP-like Dendra proteins Dendra and Dendra2, CRY2-CIBN, etc. NA-cleaving domains may advantageously also be included in the fusion polypeptides, to trigger cleave of the NA cargo. Non-limiting examples of NA cleaving domains include endonucleases such as Cas6, Cas13, engineered PUF nucleases, site specific RNA nucleases etc. Furthermore, the fusion polypeptides of the present invention may also include endosomal escape domains to drive endosomal escape and thereby enhance the bioactive delivery of the EV *per se* and the EV NA cargo molecule.

Another strategy for enhancing delivery is to target the EVs to cells, tissues, and/or organs or other bodily compartments. Targeting can be achieved by a variety of means, for instance the use of targeting peptides. Such targeting peptides may be anywhere from a few amino acids in length to several 100s of amino acids in length, e.g. anywhere in the interval of 3-100 amino acids, 3-30 amino acids, 5-25 amino acids, e.g. 7 amino acids, 12 amino acids, 20 amino acids, etc. Targeting peptides of the present invention may also include full length proteins such as receptors, receptor ligands, etc. Furthermore, the targeting peptides as per the present invention may also include antibodies and antibody derivatives, e.g. monoclonal antibodies, single chain variable fragments (scFvs), other antibody domains, etc.

The present invention utilizes polynucleotide constructs encoding for the fusion polypeptides as per the present invention. The polynucleotide constructs may be present in various different forms and/or in different vectors. For instance, the polynucleotides may be essentially linear, circular, and/or has any secondary and/or tertiary and/or higher order structure. Furthermore, the present invention also relates to vectors comprising the polynucleotides, e.g. vectors such as plasmids, any circular DNA polynucleotide for instance a plasmid, mini-circles, viruses such as adenoviruses, adeno-associated viruses, lentivirus, mRNAs, naked viral genomes, and/or modified mRNAs. The EV-producing cells may be genetically modified with the at least one polynucleotide construct using essentially any non-viral or viral method for introducing a polynucleotide into a cell. Suitable methods include transfection using a polycationic reagent such as PEI, lipid-based transfection reagents such as Lipofectamine (RTM), lentiviral transduction, CRISPR-Cas guided insertion, Flp-In system, transposon system, electroporation, DEAE-Dextran transfection, and calcium phosphate transfection. The choice of method for introducing the polynucleotide into an EV-producing cell will depend on various parameters, including choice of cell source, the nature and characteristics of the polynucleotide vector (e.g. if the vector is a plasmid or a minicircle or e.g. a linear DNA polynucleotide or an mRNA), and the level of compliance and control needed. Similarly, immortalization of EV-producing cells to create stable cell lines can be achieved using techniques that are well known in the art of cell line development, including hTERT-mediated immortalization, transcription factor immortalization, E1/E2 immortalization or other virusmediated immortalization techniques, etc.

The polynucleotide constructs as per the present invention may further comprise one or more sites or domains for imparting particular functionality into the polynucleotide. For example, the stability of the polynucleotide constructs can be enhanced through the use of stabilizing domains, such as additional polyA tails or stem loops, and the polynucleotide construct may also be controlled by particular promotors which may optionally be cell-type specific, inducible promotors, linkers, etc. A PolyA tail may optionally be inserted upstream of the Cas6 or Cas13 cut site so as to result in cleavage of mRNAs which retain the stabilizing PolyA tail.

The present invention further relates to various methods for producing EVs. Such methods may comprise the steps of (i) introducing into an EV-producing cell at least one polynucleotide construct encoding for an NA cargo molecule of interest, (ii) introducing into an EV-producing cell at least one polynucleotide construct encoding for PABP, and (iii) expressing in the EV-producing cell the products encoded for by the polynucleotide constructs, and (iii) collecting from the EV-producing cell the EVs that are being produced, which comprises the NA cargo molecule and the PABP . In certain embodiments, a single polynucleotide construct encoding for both the NA cargo molecule and for PABP is used whereas in other embodiments more than one polynucleotide construct is employed. Without wishing to be bound by any theory, it is surmised that the EV-producing cell into which a polynucleotide construct has been introduced (either transiently or stably, depending on the purpose and use of the EVs) produces EVs (such as exosomes) that comprise the PABP protein encoded for by the polynucleotide as well as the NA cargo molecule, which is stabilized by the PABP protein and loaded into the EVs secreted from the EV-producing cells. In alternative embodiments, PABP may be comprised in a fusion polypeptide encoded for by a corresponding polynucleotide construct. As abovementioned, such a fusion protein would normally comprise at least one exosomal polypeptide, at least one NA-binding domain (either a domain of or essentially a complete NA-binding protein), and at least one PABP protein, wherein the fusion protein is intended to enhance loading into EVs of the NA cargo molecule in question, via interaction between the NA-binding domain and the NA and via NA stabilisation mediated by PABP.

Once the EVs have been secreted from the EV-producing cells, the EVs may then optionally be collected, typically from the cell culture media, and optionally further purified before being put to a particular use. Typically, a single EV comprises several copies of the NA cargo molecule but a single EV may also comprise more than one type of NA drug cargo molecule. As a non-limiting example of EVs comprising more than one type of NA drug cargo, a single EV (i.e. a population of a single type of EVs) may comprise e.g. an mRNA drug cargo molecule and a gRNA NA drug cargo molecule. In a preferred embodiment the method further comprises introduction and expression of a polynucleotide construct encoding the exosomal protein-NA-binding domain fusion protein and/or the TIF.

In a further aspect, the present invention relates to *in vitro* methods for intracellular delivery of at least one NA cargo molecule. Such methods may advantageously be carried out *in vitro* and/or *ex vivo.* The methods may comprise the steps of contacting a target cell with at least one EV as per the present invention, or more commonly a population of EVs as per the present invention. Furthermore, the methods for delivery of NA cargo molecules as per the present invention may also comprise introducing into a cell present in any biological system (such as a human being) polynucleotides encoding for the NA cargo molecule, PABP and optionally the fusion polypeptides herein.

In additional aspects, the present invention relates to pharmaceutical compositions comprising either one of more of the following components: (i) at least one EV as described herein and/or (ii) at least one population of EVs as described herein, typically formulated with a pharmaceutically acceptable excipient, carrier and/or diluent or similar. Furthermore, the present invention also pertains to the (i) at least one EV as described herein and/or (ii) at least one population of EVs as described herein, and the (iii) above-mentioned pharmaceutical composition, for use in medicine, more specifically for use in the prophylaxis and/or treatment and/or alleviation of a variety of diseases. Non-limiting examples of diseases and conditions include the following non-limiting examples: Crohn's disease, ulcerative colitis, ankylosing spondylitis, rheumatoid arthritis, multiple sclerosis, systemic lupus erythematosus, sarcoidosis, idiopathic pulmonary fibrosis, psoriasis, tumor necrosis factor (TNF) receptorassociated periodic syndrome (TRAPS), deficiency of the interleukin-1 receptor antagonist (DIRA), endometriosis, autoimmune hepatitis, scleroderma, myositis, stroke, acute spinal cord injury, vasculitis, Guillain-Barré syndrome, acute myocardial infarction, ARDS, sepsis, meningitis, encephalitis, liver failure, non-alcoholic steatohepatitis (NASH), non-alcoholic fatty liver disease (NAFLD), kidney failure, heart failure or any acute or chronic organ failure and the associated underlying etiology, graft-vs-host disease, Duchenne muscular dystrophy and other muscular dystrophies, urea cycle disorders such as N-Acetylglutamate synthase deficiency, carbamoyl phosphate synthetase deficiency, ornithine transcarbamoylase deficiency, citrullinemia (deficiency of argininosuccinic acid synthase), argininosuccinic aciduria (deficiency of argininosuccinic acid lyase), argininemia (deficiency of arginase), hyperornithinemia, hyperammonemia, homocitrullinuria (HHH) syndrome (deficiency of the mitochondrial ornithine transporter), citrullinemia II (deficiency of citrin, an aspartate glutamate transporter), lysinuric protein intolerance (mutation in y+L amino acid transporter 1, orotic aciduria (deficiency in the enzyme uridine monophosphate synthase UMPS), all of the lysosomal storage diseases, for instance Gaucher disease type I, II and/or III, Fabry's disease, MPS I, II (Hunter syndrome), III and IV, Niemann-Pick disease type A, B, and C, Pompe disease, cystinosis, etc., neurodegenerative diseases including Alzheimer's disease, Parkinson's disease, GBA associated Parkinson's disease, Huntington's disease and other trinucleotide repeat-related diseases, dementia, ALS, cancer-induced cachexia, anorexia, diabetes mellitus type 2, and various cancers. Virtually all types of cancer are relevant disease targets for the present invention, for instance, Acute lymphoblastic leukemia (ALL), Acute myeloid leukemia, Adrenocortical carcinoma, AIDS-related cancers, AIDS-related lymphoma, Anal cancer, Appendix cancer, Astrocytoma, cerebellar or cerebral, Basal-cell carcinoma, Bile duct cancer, Bladder cancer, Bone tumor, Brainstem glioma, Brain cancer, Brain tumor (cerebellar astrocytoma, cerebral astrocytoma/malignant glioma, ependymoma, medulloblastoma, supratentorial primitive neuroectodermal tumors, visual pathway and hypothalamic glioma), Breast cancer, Bronchial adenomas/carcinoids, Burkitt's lymphoma, Carcinoid tumor (childhood, gastrointestinal), Carcinoma of unknown primary, Central nervous system lymphoma, Cerebellar astrocytoma/Malignant glioma, Cervical cancer, Chronic lymphocytic leukemia, Chronic myelogenous leukemia, Chronic myeloproliferative disorders, Colon Cancer, Cutaneous T-cell lymphoma, Desmoplastic small round cell tumor, Endometrial cancer, Ependymoma, Esophageal cancer, Extracranial germ cell tumor, Extragonadal Germ cell tumor, Extrahepatic bile duct cancer, Eye Cancer (Intraocular melanoma, Retinoblastoma), Gallbladder cancer, Gastric (Stomach) cancer, Gastrointestinal Carcinoid Tumor, Gastrointestinal stromal tumor (GIST), Germ cell tumor (extracranial, extragonadal, or ovarian), Gestational trophoblastic tumor, Glioma (glioma of the brain stem, Cerebral Astrocytoma, Visual Pathway and Hypothalamic glioma), Gastric carcinoid, Hairy cell leukemia, Head and neck cancer, Heart cancer, Hepatocellular (liver) cancer, Hodgkin lymphoma, Hypopharyngeal cancer, Intraocular Melanoma, Islet Cell Carcinoma (Endocrine Pancreas), Kaposi sarcoma, Kidney cancer (renal cell cancer), Laryngeal Cancer, Leukemias ((acute lymphoblastic (also called acute lymphocytic leukemia), acute myeloid (also called acute myelogenous leukemia), chronic lymphocytic (also called chronic lymphocytic leukemia), chronic myelogenous (also called chronic myeloid leukemia), hairy cell leukemia)), Lip and Oral, Cavity Cancer, Liposarcoma, Liver Cancer (Primary), Lung Cancer (Non-Small Cell, Small Cell), Lymphomas, AIDS-related lymphoma, Burkitt lymphoma, cutaneous T-Cell lymphoma, Hodgkin lymphoma, Non-Hodgkin, Medulloblastoma, Merkel Cell Carcinoma, Mesothelioma, Metastatic Squamous Neck Cancer with Occult Primary, Mouth Cancer, Multiple Endocrine Neoplasia Syndrome, Multiple Myeloma/Plasma Cell Neoplasm, Mycosis Fungoides, Myelodysplastic/Myeloproliferative Diseases, Myelogenous Leukemia, Chronic Myeloid Leukemia (Acute, Chronic), Myeloma, Nasal cavity and paranasal sinus cancer, Nasopharyngeal carcinoma, Neuroblastoma, Oral Cancer, Oropharyngeal cancer, Osteosarcoma/malignant fibrous histiocytoma of bone, Ovarian cancer, Ovarian epithelial cancer (Surface epithelial-stromal tumor), Ovarian germ cell tumor, Ovarian low malignant potential tumor, Pancreatic cancer, Pancreatic islet cell cancer, Parathyroid cancer, Penile cancer, Pharyngeal cancer, Pheochromocytoma, Pineal astrocytoma, Pineal germinoma, Pineoblastoma and supratentorial primitive neuroectodermal tumors, Pituitary adenoma, Pleuropulmonary blastoma, Prostate cancer, Rectal cancer, Renal cell carcinoma (kidney cancer), Retinoblastoma, Rhabdomyosarcoma, Salivary gland cancer, Sarcoma (Ewing family of tumors sarcoma, Kaposi sarcoma, soft tissue sarcoma, uterine sarcoma), Sézary syndrome, Skin cancer (nonmelanoma, melanoma), Small intestine cancer, Squamous cell, Squamous neck cancer, Stomach cancer, Supratentorial primitive neuroectodermal tumor, Testicular cancer, Throat cancer, Thymoma and Thymic carcinoma, Thyroid cancer, Transitional cell cancer of the renal pelvis and ureter, Urethral cancer, Uterine cancer, Uterine sarcoma, Vaginal cancer, Vulvar cancer, Waldenström macroglobulinemia, and/or Wilm's tumor.

The EVs as per the present invention may be administered to a human or animal subject via various different administration routes, for instance auricular (otic), buccal, conjunctival, cutaneous, dental, electro-osmosis, endocervical, endosinusial, endotracheal, enteral, epidural, extra-amniotic, extracorporeal, hemodialysis, infiltration, interstitial, intra-abdominal, intra-amniotic, intra-arterial, intra-articular, intrabiliary, intrabronchial, intrabursal, intracardiac, intracartilaginous, intracaudal, intracavernous, intracavitary, intracerebral, intracerebroventricular, intracisternal, intracorneal, intracoronal (dental), intracoronary, intracorporus cavernosum, intradermal, intradiscal, intraductal, intraduodenal, intradural, intraepidermal, intraesophageal, intragastric, intragingival, intraileal, intralesional, intraluminal, intralymphatic, intramedullary, intrameningeal, intramuscular, intraocular, intraovarian, intrapericardial, intraperitoneal, intrapleural, intraprostatic, intrapulmonary, intrasinal, intraspinal, intrasynovial, intratendinous, intratesticular, intrathecal, intrathoracic, intratubular, intratumor, intratym panic, intrauterine, intravascular, intravenous, intravenous bolus, intravenous drip, intraventricular, intravesical, intravitreal, iontophoresis, irrigation, laryngeal, nasal, nasogastric, occlusive dressing technique, ophthalmic, oral, oropharyngeal, other, parenteral, percutaneous, periarticular, peridural, perineural, periodontal, rectal, respiratory (inhalation), retrobulbar, soft tissue, subarachnoid, subconjunctival, subcutaneous, sublingual, submucosal, topical, transdermal, transmucosal, transplacental, transtracheal, transtympanic, ureteral, urethral, and/or vaginal administration, and/or any combination of the above administration routes, which typically depends on the disease to be treated and/or the characterstics of the EVs, the NA cargo molecule in question, or the EV population as such.

### Examples

### Materials and Methods

### Construct design and cloning

Two different types of DNA constructs have been designed to express PABP from a mammalian expression vector. The first one involve the cloning of ORF of PABP fused to two HA tags and the second design involve the cloning of the ORF of PABP separated from the exosomal polypeptide fused to the RNA binding domain by a P2A. After translation, the P2A sequence is subjected to a cleavage reaction producing to different poly peptide, the exosomal protein fused to the RNA binding domain and PABP. This allows the expression of the two proteins of interest from the same plasmid. The vector chosen is the FC5501 -A, a donor vector from the PHiC31 integrase system (SBI), which allows the transient transfection analysis of the cloned proteins, as well as the stable integration of the insert in the genome when cotransfecting this plasmid in the presence of the plasmid expressing the phiC31 integrase. The ORFs were typically generated by synthesis. Briefly, for the HA-PABP constructs, both the insert and the plasmid were digested with the enzymes EcoRI and Mlul as per manufacturers instruction (NEB). Restricted, purified DNA fragments have been ligated together using T4 ligase as per manufacturers instruction (NEB). Successful ligation events have been selected by bacterial transformation on ampicillin-supplemented plates. Restriction analysis was performed on several colonies and two of the ones containing the right size insert were sent for sequencing to verify the correct sequence. The required amount of the plasmid for the following experiments was generated by 'maxi-prep', as per manufacturers instructions.

Due to the larger size of the insert for the P2A containing fragment, the cloning was performed in two consecutive steps. First, a synthesized fragment containing the exosomal polypetide fused to the RNA binding domain, was cut with EcoRI and Nhel as well as the recipient plasmid. After ligation, the successful ligation events were analysed by restriction to contain the right size of the insert and sent for sequencing. A plasmid containing the correct sequence when then cut with Nhel and Mlul as well as the second synthesized fragment encoding for the P2A_PABP. The same strategy of ligation and selection of the correct clones as described earlier was followed, as well as for the generation of a larger amount of the plasmid for the following experiments.

Various NA-binding domains and variants thereof (e.g. PUF, mutated PUF, PUFx2, Cas6, mutated Cas6, Cas13, mutated Cas13, MS2 etc.) have been assessed, in combination with several exosomal polypeptides (such as CD81, CD63, CD9, syntenin, syndecan, Alix, CD133, etc.). ORFs were typically generated by synthesis and cloned into the mammalian expression vector pSF-CAG-Amp. Briefly, synthesized DNA and vector plasmid were digested with enzymes Notl and Sall as per manufacturers instruction (NEB). Restricted, purified DNA fragments were ligated together using T4 ligase as per manufacturers instruction (NEB). Successful ligation events were selected for by bacterial transformation on ampicillin-supplemented plates. Plasmid for transfection was generated by 'maxi-prep', as per manufacturers instruction.

### Cell culture, transfection and EV uptake

### Cell culture

Depending on the experiment design and assays, transient transfection and EVs production was carried out in conventional 2D cell culture. In other cases, stable cell lines were stablished using the PhiC31 integration system from SBI.

In the case of transient transfection, HEK293 cells stably expressing the mRNA of interest with or without stably expressing the exosomal protein fused to the RNA binding domain (or no bind control), were seeded into 15 cm dishes and left overnight in serum-containing DMEM, as recommended by ATCC. The following day, cells were transiently transfected with the corresponding plasmid DNA (expressing PABP, and when required, in combination with the plasmid expressing the exosomal protein fused to the RNA binding domain or no bind control) using lipofectamine2000, as per manufacture instructions. Briefly, the plasmid(s) DNA was incubated with the required amount of Lipofectamine2000 in Opti-MEM for 5 minutes at room temperature. After incubation, lipofectamine-DNA complexes were gently added to the cells. 24h after transfection, serum-containing DMEM was replaced by Opti-MEM for 48h prior to proceeding with media and cells harvesting.

For the generation of stable cells lines expressing the different combination of exosomal polypeptide fused to the RNA binding domain or no bind control with P2A and PABP or, the plasmid expressing HA2-PABP, HEK293 cells were transfected with the corresponding plasmid DNA as described earlier in the presence of and additional plasmid expressing the phiC31 integrase. 48h after transfection, the cells were passaged, seeded in a 6 well plate and treated with different amounts of the corresponding antibiotic to select for the cells which have incorporated the insert in the genome. After 2-3 weeks under selection, the cells were collected, and the expression of the integrated protein was analysed by western blot and/or flow cytometry. In addition to HEK293 cells, the same protocols with minor adaptations were employed for engineering and development of suspension HEK293 cells, serum free HEK293 cells, Wharton's jelly MSCs, bone marrow-derived MSCs, amnion epithelial cells, placentaderived MSCs and other cell sources.

### EV purification

Depending of the experiment, either 48h after transient transfection of HEK293T or 48h after the seeding of stable cells lines, the media was changed to OPTI-MEM to boost EVs production. 48h later, the conditioned media was harvested and the EVs were isolated using a variety of methods, typically a combination of filtration such as TFF, size exclusion chromatography or Ultracentrifugation and/or bead-elute liquid chromatography. Typically, EV containing media was subjected to differential centrifugation 5' at 700g and then 10' at 2000g to remove cells and debris respectively. Then, a 0.22 µm filtration is performed to remove any remaining large impurity. After an ultra-centrifugation cycle at 100.000g for 90', the supernatant is removed, and the resulting pellet resuspended in 0.22 µm filtered PBS 1X. A second round of centrifugation is normally carried out at 100.000g for 90' and the resulting EVs pellet is then resuspended in 200 µL of 0.22 µm filtered PBS.

When large volumes of conditioned media are required, after the differential centrifugation and 0.22um filtration, the conditioned media is dialfiltered and concentrated using the Vivaflow 50R tangencial flow (TFF) device (Sartorius) with 100 KDa cutoff filters. The preconcentrated medium was subsequently loaded onto the bead-eluted columns (HiScreen (RTM) or HiTrap (RTM) Capto Core 700 columns, GE Heathcare life sciences), connected to an AKTAprime (RTM) plus of AKTA Pure 25 (RTM) chromatography system (GE Healthcare Life Sciences). Flow rate setting for column equilibration, sample loading and column cleaning procedure in place were chose according to the manufacturer's instructions. The sample was collected according to the UV absorbance chromatogram.

### Uptake assay

By way of example, 10⁵ Huh7 cells were seeded in a 24well plate in serum-containing DMEM. 24h after seeding, the media is removed and replace by 500ul of serum-free OPTI-MEM containing the desired amount of, for instance, HEK293 EVs (ranging from 10¹⁰ to 10⁸). The media containing EVs was also added to an extra well without cells to account for the background of protein loaded into EVs. 16h after treatment, the supernatant is collected, cells washed in PBS 1X and detached by trypsin. Supernatant and PBS wash of each condition is pooled together and the cells are counted and cell viability assessed by Countess II FL Automated Cell Counter.

The RNA from the cells is then extracted and the presence of the RNA of interest analysed by RT-qPCR. The expression of proteins is analysed by Western blot and/or Luciferase analysis, as described below.

### Assays and analytics

Western blot is a highly convenient analytical method to evaluate the enrichment of proteins in EVs and the expression in cells. Briefly SDS-PAGE was performed according to manufacturer's instruction (Invitrogen, Novex PAGE 4-12% gels). Protein concentration of the cell lysates was assessed using the Pierce BCA Protein Assay kit as per manufacturer instructions, and 40ug were loaded in the gel. Generally, 1× 10¹⁰ EVs were loaded. Proteins from the SDS-PAGE gel have been transferred to PVDF membrane according to manufacturer's instruction (Immobilon (RTM), Invitrogen). Membranes have been blocked in Odyssey blocking buffer (Licor) for 1h at room temperature and probed with primary antibodies according to supplier's instruction. Primary antibodies against CD63, PABP, HA, Tubulin, and Alix were obtained from purchased form Abcam. Nanoluc antibody was kindly provided by Promega. Secondary antibodies from Licor have been used following the manufacturer's instructions. Molecular probes visualized at 680 and 800 nm wavelengths.

For EV number and size determination, nanoparticle tracking analysis (NTA) was performed with a Zetaview PMX-120 (Particle Metrix) instrument equipped with analytical software. Alternatively nanoparticle tracking analysis (NTA) was performed with a NanoSight (RTM) instrument equipped with analytical software. For recordings on the NanoSight (RTM), a camera level of 13 or 15 and automatic function for all post-acquisition settings were used. Electron microscopy and fluorescence microscopy were frequently used to validate and assess EV morphology and size.

The extraction of RNA from the EVs to analyse the loading was carried out using Direct-zol^{™} RNA MicroPrep kit (Zymo Research). F or the extraction of RNA from the cells, first the cells were lysed using TRI Reagent (Thermo Fisher). After solubilization, total RNA was isolated adding chloroform. The aqueous phase, containing the RNA, was mixed with the same volume of 100% Ethanol and subsequently the RNA extraction was carried using the Direct-zol RNA Miniprep kit following the manufacturer's protocol (ZYMO RESEARCH). A DNase treatment step was performed to remove all the traces of genomic and plasmid DNA. The RNA was finally quantified with a Spectrophotometer and 100 ng was used in a Retro transcription reaction to generate the cDNA, either with random primers, oligo dT or a transcript specific primer, using the Reverse Transcription kit High-Capacity cDNA Reverse Transcription Kit (Applied Biosystems). 2ng of the newly synthesized cDNA were used to perform a quantitative PCR using the PowerUp SYBR Green Master Mix (ThermoFisher Scientific) and StepOnePlus qRT-PCR machine (ThermoFisher Scientific) according to manufacturer instruction.

Absolute copies per cell/EVs of the target transcripts were calculated using a standard curve of each transcript amplicon.

For the luciferase assay, cell pellets were lysed with 50ul of Passive Lysis Buffer 1X (Promega) and the luminescence measured following the Nano-Glo^{®} Luciferase Assay System. Together with the cell lysates, luminescence has been assessed in the supernatant of the cell culture, the PBS wash as well as in the EVs supernatant.

### Examples

### Example 1

Bone marrow-derived MSCs were cultured in conventional tissue culture flasks and transiently transfected using PEI transfection to enable loading and expression of mRNA cargo molecules and fusion polypeptide constructs. Figure 2 shows loading in EVs obtained from the BM-MSCs of mRNA cargo molecules encoding NanoLuc (RTM) and p21. Active NA cargo molecule loading was achieved using engineering of fusion polypeptide constructs comprising CD63 as the exosomal polypeptide and PUF or Cas6 as the NA-binding domains, in combination with PABP either as part of the fusion protein construct or encoded as a separate protein. The experiment also included varying numbers of binding sites for the NA-binding domains on the NA cargo molecule, namely 0, 3 and 6 binding sites, which were inserted in different places to the 3' and/or 5' flanks of the coding region.

The MSC-EVs were purified using a sequential combination of TFF and SEC. Expression of only the exosomal polypeptide CD63 did not result in loading of any mRNA into the EVs (right set of columns in the graph in Figure 2). Expression of fusion polypeptides comprising PUF (left set of columns: two PUFs domain flanking CD63 both N terminally and C terminally, i.e. 4 PUF constructs in total) (second set of columns from left: one PUF domain flanking CD63 both N terminally and C terminally) and mutated Cas6 (second from right) did result in significant mRNA loading of both NanoLuc (RTM) and p21 mRNAs upon expression in the EV source cells. The loading of NanoLuc (RTM) was overall more efficient than the loading of p21, with up to around 45 copies of mRNA per EV.

### Example 2

Expression of NanoLuc (RTM) as a reporter system in target Huh7 cells after HEK EV-mediated delivery of a NanoLuc (RTM) mRNA. HEK293T cells were stably transduced to express various fusion polypeptide constructs for loading of reporter NanoLuc (RTM) NA cargo into EVs. The NanoLuc (RTM) NA cargo molecule was engineered to comprise 0, 3, or 6 binding sites for the NA-binding domains comprising the fusion polypeptide constructs, in this case PUFx2-CD63- PUFx2 (two PUF NA-binding polypeptides inserted both N terminally and C terminally of the exosomal polypeptide CD63), PUF-CD63-PUF, and Cas6-CD9-Cas6 (Figure 3).

Post purification of the HEK-derived EVs based on TFF combined with bead-elute LC the EVs were added to HEK cells in at optimal concentration, which for this assay was 10^7 EVs per well in 6-well plates of Huh7 target cells. The Y axis of Figure 3 shows relative light (luminescence) units (RLU) normalized over µg of protein, indicating enhanced delivery and/or translation with increasing numbers of binding sites using the different fusion polypeptide constructs. Figure 3, therefore, demonstrates that the present invention provides EVs which are capable of delivering bioactive mRNA to cells which is then successfully translated by these cells. This is a significant advantage of the present invention over the prior art which is only able to load EVs with RNA but is not able to deliver those RNAs to the cytosol of target cells to be actively translated.

### Example 3

Figure 5 demonstrates that when HEK293 cells are transiently transfected with HA-PABP and CD63-PUFeng and stably express Nanoluc mRNA both PAPB and the fusion protein CD63-PUFeng present in the cell lysate as well as the extracellular vesicles purified from the conditioned media.

In Figure 5A identical amounts of HEK293 cells lysates were transiently transfected with a pcDNA3 plasmid with cDNA of PABP with two HA tags in the N-terminal, and with a pLex plasmid with the cDNA for CD63-PUFeng, or transfected with the control plasmids lacking the PUFeng nucleic acid binding domain. The western blots shown were immunoblotted with antibodies against PABP, CD63, Nanoluc and Tubulin (used as loading control). Figure 5A shows that PABP is overexpressed in response to transfection of the cells with the PABP construct and that the CD63-PUFeng fusion protein is also expressed in cells when this construct is transfected into cells. It is clear from well 4 that overexpression of both PABP and CD63-PUFeng is possible in the same cell type.

Figure 5B shows the same number of EVs purified from the conditioned media of the transiently transfected cells (cells as described above) which were immunoblotted with antibodies against PABP, CD63, Nanoluc and Alix (used as loading control). The results of Figure 5B show that cells transfected as described above with different constructs produce EVs which also contain the same overexpressed levels of PAPB and/or CD63-PUFeng as the cells from which they are produced.

### Example 4 - Evidence for Increased mRNA Levels in EVs in the Presence of PABP

Figure 6 demonstrates that over-expression of PABP in EV producer cells increases the number of molecules of mRNA loaded into EVs. The inventors observe a 180% increase in the number of mRNA molecules per producer cell when PABP is overexpressed (data not shown). The EVs from these producer cells were then purified from the conditioned media of the HEK293T stable cell line which expresses the NanoLuc mRNA with PUFeng interacting sites in the 3'UTR, and is transiently transfected with the engineered exosomal protein CD63 fused to the RNA binding domain PUFeng in the C-terminal and -/+ transient transfection of a plasmid expressing HA2-PABP. After EV purification the RNA was extracted and reverse transcribed using an oligodT primer. An amplicon corresponding to a region in the 5' end of the Nanoluc mRNA was then quantified using qPCR absolute quantification with a standard curve.

The table in Figure 6 shows the percentage increases in the number of Nanoluc mRNA molecules per 1*10⁶ EVs detected. Fold enrichments are calculated as the ratio between the number of molecules loaded into EVs in the presence of PABP, and the number of molecules loaded into EVs without PABP, for both, the bind condition (CD63-PUFeng) and the no-bind control (CD63-MS2). The loading of Nanoluc was more efficient in the presence of PABP .

Figure 6 shows that merely the presence of PABP alone induces a 300% increase in the number of mRNA molecules per EV (i.e. stabilization of mRNA loaded by passive loading alone) and PAPB in combination with the CD63-PUFeng loading construct leads to a 1000% increase in the number of mRNA molecules loaded into EVs produced by the producer cells. The presence of PABP stabilizing the mRNA and thus increasing the half-life or half-life of the mRNA in the producer cells is shown to increase the loading of mRNA into the EVs both with and without the additional beneficial effect of the fusion protein loading construct.

### Example 5 - Evidence of Translation of mRNA in Recipient Cells

Figure 7 demonstrates that the number of Nanoluc mRNA molecules delivered to Huh7 recipient cells (by treatment with mRNA loaded EVs) is higher in the presence of PABP and also leads to an increase in the RLUs detected in the cell lysates as a result of an increase in translation of the delivered mRNA molecules.

The uptake experiment shown in Figure 7 was carried out using EVs containing Nanoluc mRNA +/- PABP. The Y axis depicts the total RLUs (Relative Luminescence Units) released by the NanoLuc protein when translated normalized to the µg of proteins in the cell lysate and to total RLUs measured in the EVs. The RNA from Huh7 was extracted and reverse transcribed using an oligodT primer. An amplicon corresponding to a region in the 5' end of the Nanoluc mRNA was then quantified using qPCR and absolute quantification with a standard curve. This shows that the number of mRNAs delivered to recipient cells is significantly increased by the presence of PABP as a stabilizer of the mRNA both with and without the additional effect of the fusion loading construct indicating that the PABP is not only stabilizing the mRNA in the producer cells and the whilst carried in the EVs produced but also continues to stabilize the mRNA once the EVs are taken up into the recipient cells and the mRNA is released into the recipient cells.

Additionally, the RLU data shows that the increase in number of mRNA number detected in the recipient cells corresponds to an increase in level of translation of the cargo mRNA in the recipient cell. This demonstrates the ability of the present invention, by employing PABP, is able to deliver more bioactive mRNA molecules to recipient cells and that the increased stability of the mRNA delivered results in significantly increased levels of translation of the cargo mRNA into the desired therapeutic protein in the recipient cell.

Figure 8 shows a repeat of the uptake experiment shown in Figure 7 (method as described in Example 5 except that the EVs were delivered at 10x dilution). Dilution was used in order to show more clearly the effect of the addition of PABP on the levels of translation of the delivered mRNA. Dilution of the EV sample applied to the recipient cells allows clearer distinction of the levels of mRNA translation in recipient cells because it prevents saturation of the newly translated NanoLuc signal by NanoLuc protein which has been passively loaded into the EVs alongside the mRNA. The number of NanoLuc mRNA molecules delivered to Huh7 recipient cells is higher in the presence of PABP and also leads to an increase (7 fold) in the RLUs detected in the cell lysates as a result of an increase of translation of the delivered mRNA molecules.

Similar to the data in Figure 7, the RLU data in Figure 8 shows that the increase in number of mRNA detected in the recipient cells corresponds to an increase in level of translation of the cargo mRNA in the recipient cell. This demonstrates the ability of the present invention, by employing PABP, to deliver more bioactive mRNA molecules to recipient cells and that the increased stability of the mRNA delivered results in significantly increased levels of translation of the cargo mRNA into the desired therapeutic protein in the recipient cell.

### Example 6 - Evidence of Stabilization of Passively Loaded Nucleic Acids

Furthermore, the data in figures 6-8 shows that the presence of PABP also improves the loading of passively loaded mRNAs. Figure 6 shows that merely the presence of PABP alone (without the use of an NA binding protein such as PUF, i.e. the "no bind" examples) induces a 300% increase in the number of mRNA molecules per EV. Fold enrichments are calculated as the ratio between the number of molecules loaded into EVs in the presence of PABP, and the number of molecules loaded into EVs without PABP, for both. The loading of Nanoluc was more efficient in the presence of PABP without any assistance from the exosomal protein-NA binding domain fusion protein.

The "no bind" data in Figures 7 and 8 show that the passively loaded mRNA which has been stabilized by the added PABP results in increased translation of the mRNA in recipient cells showing that addition of PABP increases the bioactive delivery of passively loaded mRNA. Figures 7 and 8 show that when mRNA is loaded passively into EVs (without the use of the NA binding protein) the presence of PABP in these cells is capable of stabilization of mRNA loaded by this passive loading. In particular the data in Figure 8 show a 4 fold increase in the levels of translation in recipient cells. The presence of PABP stabilizes the mRNA thus increasing the half-life of the mRNA in producer cell, whilst it is passively loaded into the EV and also when delivered to recipient cell.

### Example 7 - Stabilization of Exogenously Loaded Nucleic acids

The data in Figures 6-8 also show that it would be possible to improve the delivery of nucleic acids which are exogenously added to EVs by increasing their stability.

The present invention relates to an EV comprising at least one polyA binding protein (PABP) or a fragment or domain thereof and at least one NA cargo molecule comprising a contiguous stretch of adenine nucleotides, wherein the NA cargo molecule is exogenously loaded into the EV. The NA cargo molecule may naturally comprise the contiguous stretch of adenine nucleotides (i.e. the polyA region) or the polyA region may be introduced into said NA cargo molecule to increase its stability.

Figures 6-8 show that it would be possible to stabilise mRNA or any other NA cargo comprising a polyA tail that is exogenously added to EVs so as to deliver increased levels of that NA cargo in an improved bioactive state by stabilizing the exogenous NA cargo using PABP.

As discussed above in relation to Example 6 the "no bind" data in Figures 6-8 show that the presence of PABP is also able to stabilize free NA cargos (i.e. cargo that is not bound to an RNA binding protein). The "no bind" data in figures 6-8 show that the passively loaded mRNA which has been stabilized by the added PABP results in a 300% increase in the number of mRNA molecules per EV and also increased translation of the mRNA in recipient cells showing that addition of PABP increases the bioactive delivery of passively loaded mRNA. Figures 7 -8 show that when mRNA is loaded passively into EVs (without the use of the RNA binding protein) the presence of PABP in these cells is capable of stabilization of mRNA loaded by this passive loading. The data in Figure 8 shows a 4 fold increase in the levels of translation in recipient cells. The presence of PABP stabilizes the mRNA thus increasing the half-life of the mRNA whilst loaded in the EV and also when delivered to recipient cell.

This data indicates that it would be possible to produce EVs loaded with stabilized NA cargo by a method comprising a) production of EVs from a producer cell line, b) purification of EVs by any known method, c) production and purification of an NA cargo comprising a contiguous stretch of adenine nucleotides or synthesis of artificial NA cargo comprising a contiguous stretch of adenine nucleotides, d) mixing this exogenous NA cargo with a quantity of a PABP in order to stabilize the NA cargo, and e) loading said stabilized NA cargo exogenously into EVs by any known method. Known loading methods include electroporation, transfection with transfection reagents (such as cationic transfection agents, for instance lipofectamine (RTM)), or loading by means of a cell penetrating peptide (CPP).

## Claims

1. An extracellular vesicle (EV) comprising at least one polyA binding protein (PABP) or a fragment or domain thereof capable of binding to a contiguous stretch of adenosine bases, at least one nucleic acid (NA) cargo molecule comprising a contiguous stretch of adenine nucleotides, and at least one fusion polypeptide comprising at least one NA-binding domain and at least one exosomal polypeptide.

2. The EV according to claim 1:
(a) wherein PABP is comprised in the fusion polypeptide; and/or
(b) wherein the at least one NA-binding domain is one or more mRNA binding proteins, pre-rRNA-binding proteins, tRNA-binding proteins, small nuclear or nucleolar RNA-binding proteins, non-coding RNA-binding proteins, miRNA-binding proteins, shRNA-binding proteins.

3. The EV according to any one of the preceding claims, wherein the at least one NA cargo molecule:
(a) is transported into the EV by binding to the fusion protein;
(b) comprises at least one binding site for the NA binding domain;
(c) comprises at least one cleavage site between the NA binding site and the domain of the NA cargo that has biologic, therapeutic and/or prophylactic activity;
(d) is selected from the group comprising shRNA, miRNA, mRNA, gRNA, pri-miRNA, pre-miRNA, circular RNA, piRNA, tRNA, rRNA, snRNA, IncRNA, ribozymes, mini-circle DNA, and/or plasmid DNA; and/or
(e) is an mRNA molecule which encodes for a therapeutic and/or prophylactic protein or peptide.

4. The EV according to claim 3(e) wherein the therapeutic protein or peptide is selected from the group comprising: antibodies, intrabodies, single chain variable fragments, affibodies, enzymes, transporters, tumor suppressors, viral or bacterial inhibitors, cell component proteins, DNA and/or RNA binding proteins, DNA repair inhibitors, nucleases, proteinases, integrases, transcription factors, growth factors, apoptosis inhibitors and inducers, toxins, structural proteins, neurotrophic factors, membrane transporters, lysosomal proteins, nucleotide binding proteins, heat shock proteins, CRISPR-associated proteins, and any fragment, domain and/or combination thereof.

5. The EV according to any one of the preceding claims, wherein the exosomal polypeptide is selected from the group comprising CD9, CD63, CD81, FLOT1, FLOT2, ALIX, ARRDC1, Syntenin-1, Syntenin-2, Lamp1, Lamp1b, TSG101, Lamp2a, Lamp2b, TSPAN8, syndecan-1, syndecan-2, syndecan-3, syndecan-4, TSPAN14, CD82, CD47, other exosomal polypeptides, and any regions fragments or combinations thereof.

6. The EV according to any one of the preceding claims, wherein the EV further comprises:
(a) at least one translation initiation factor (TIF); and/or
(b) at least one targeting moiety which targets the EV to a target cell, tissue, organ, organelle or other bodily location.

7. The EV according to any one of the preceding claims, wherein when the NA cargo molecule is an mRNA cargo molecule it further comprises:
a. secondary, tertiary and/or other structure; and/or
b. at least one stabilizing stem loop; and/or
c. at least one hybrid UTR in the 5' and/or 3' end.

8. The EV according to any one of the preceding claims wherein the EV is: an exosome, or microvesicle.

9. A population of EVs according to any one of the preceding claims.

10. The population of EVs according to claim 9 wherein:
(a) the average number of NA cargo molecules per EV throughout the population of EVs is above one per EV; and/or
(b) at least 5%, at least 10%, at least 20%, at least 50%, at least 70%, at least 75%, at least 80%, at least 85%, at least 90%, and/or at least 95% of all EVs comprise at least one NA cargo molecule.

11. A method for producing the EVs according to any one of the preceding claims, comprising:
(i) introducing into an EV-producing cell at least one polynucleotide construct encoding for a PABP protein or a fragment or domain thereof capable of binding to a contiguous stretch of adenosine bases; and
(ii) introducing into the same EV-producing cell at least one polynucleotide construct encoding at least one NA cargo molecule; and
(iii) introducing into the same EV-producing cell at least one polynucleotide construct encoding at least one fusion polypeptide comprising at least one NA-binding domain and at least one exosomal polypeptide; and
(iv) expressing in said EV-producing cell each of the PABP, the NA cargo molecule and the fusion polypeptide thereby generating said EVs.

12. The method for producing EVs according to claim 18:
(a) further comprising:
(i) introducing into the EV-producing cell at least one polynucleotide construct encoding for a translation initiation factor; and
(ii) expressing in the EV-producing cell the at least one polypeptide construct encoded for by the polynucleotide construct of (i), thereby generating said EVs;
(b) wherein the PAPB protein or fragment or domain thereof forms part of the fusion polypeptide comprising at least one NA-binding domain and at least one exosomal polypeptide; and/or
(c) wherein the PABP protein or fragment or domain thereof, the NA cargo molecule, the fusion polypeptide and/or the translation initiation factor may be encoded by the same or by different polynucleotide construct(s).

13. An *in vitro* method for intracellular delivery of at least one NA cargo molecule, comprising contacting a target cell with at least one EV according to any one of claims 1-8 and/or at least one population of EVs according to any one of claims 9-10.

14. A pharmaceutical composition comprising (i) at least one EV according to any one of claims 1-8, and/or (ii) at least one population of EVs according to any one of claims 9-10, and a pharmaceutically acceptable excipient or carrier.

15. The (i) at least one EV according to any one of claims 1-8, (ii) at least one population of EVs according to any one of claims 9-10, and/or (iii) the pharmaceutical composition according to claim 14, for use in medicine.

## Patentansprüche

1. Extrazelluläres Vesikel (EV), umfassend mindestens ein polyA-bindendes Protein (PABP) oder ein Fragment oder eine Domäne davon, das/die in der Lage ist, an einen zusammenhängenden Abschnitt von Adenosinbasen zu binden, mindestens ein Nukleinsäure-(NA)-Frachtmolekül, das einen zusammenhängenden Abschnitt von Adeninnukleotiden umfasst, und mindestens ein Fusionspolypeptid, das mindestens eine NA-bindende Domäne und mindestens ein exosomales Polypeptid umfasst.

2. EV nach Anspruch 1:
(a) wobei PABP in dem Fusionspolypeptid umfasst ist; und/oder
(b) wobei die mindestens eine NA-bindende Domäne ein oder mehrere mRNA-bindende Proteine, pre-rRNA-bindende Proteine, tRNA-bindende Proteine, kleine nukleare oder nukleoläre RNA-bindende Proteine, nicht-kodierende RNA-bindende Proteine, miRNA-bindende Proteine, shRNA-bindende Proteine ist.

3. EV nach einem der vorstehenden Ansprüche, wobei das mindestens eine NA-Frachtmolekül:
(a) durch Bindung an das Fusionsprotein in das EV transportiert wird;
(b) mindestens eine Bindungsstelle für die NA-bindende Domäne umfasst;
(c) mindestens eine Spaltstelle zwischen der NA- Bindungsstelle und der Domäne der NA-Fracht umfasst, die biologische, therapeutische und/oder prophylaktische Aktivität aufweist;
(d) ausgewählt ist aus der Gruppe, umfassend shRNA, miRNA, mRNA, gRNA, pri-miRNA, prä-miRNA, zirkuläre RNA, piRNA, tRNA, rRNA, snRNA, IncRNA, Ribozyme, Minicircle-DNA und/oder Plasmid-DNA; und/oder
(e) ein mRNA-Molekül ist, das für ein therapeutisches und/oder prophylaktisches Protein oder Peptid kodiert.

4. EV nach Anspruch 3(e), wobei das therapeutische Protein oder Peptid aus der Gruppe ausgewählt ist, umfassend: Antikörper, Intrabodies, variable Fragmente in Einzelketten, Affibodies, Enzyme, Transporter, Tumorsuppressoren, virale oder bakterielle Inhibitoren, Zellkomponentenproteine, DNA- und/oder RNA-bindende Proteine, DNA-Reparatur-Inhibitoren, Nukleasen, Proteinasen, Integrasen, Transkriptionsfaktoren, Wachstumsfaktoren, Apoptose-Inhibitoren und -Induktoren, Toxine, Strukturproteine, neurotrophe Faktoren, Membrantransporter, lysosomale Proteine, Nukleotid-bindende Proteine, Hitzeschockproteine, CRISPR-assoziierte Proteine und beliebige Fragmente, Domänen und/oder Kombinationen davon.

5. EV nach einem der vorstehenden Ansprüche, wobei das exosomale Polypeptid ausgewählt ist aus der Gruppe, umfassend CD9, CD63, CD81, FLOT1, FLOT2, ALIX, ARRDC1, Syntenin-1, Syntenin-2, Lamp1, Lamp1b, TSG101, Lamp2a, Lamp2b, TSPAN8, Syndecan-1, Syndecan-2, Syndecan-3, Syndecan-4, TSPAN14, CD82, CD47, andere exosomale Polypeptide und beliebige Regionsfragmente oder Kombinationen davon.

6. EV nach einem der vorstehenden Ansprüche, wobei das EV weiter umfasst:
(a) mindestens einen Translationsinitiationsfaktor (TIF); und/oder
(b) mindestens eine Zieleinheit, die das EV auf eine Zielzelle, ein Gewebe, ein Organ, eine Organelle oder eine andere Körperstelle ausrichtet.

7. EV nach einem der vorstehenden Ansprüche, wobei, wenn das NA-Frachtmolekül ein mRNA-Frachtmolekül ist, es weiter umfasst:
a. eine sekundäre, tertiäre und/oder andere Struktur; und/oder
b. mindestens eine stabilisierende Stamm-Schleife; und/oder
c. mindestens eine Hybrid-UTR am 5'- und/oder 3'-Ende.

8. EV nach einem der vorstehenden Ansprüche, wobei das EV: ein Exosom oder Mikrovesikel ist.

9. Population von EVs nach einem der vorstehenden Ansprüche.

10. Population von EVs nach Anspruch 9, wobei:
(a) die durchschnittliche Anzahl von NA-Frachtmolekülen pro EV in der gesamten Population von EVs über einem pro EV liegt; und/oder
(b) mindestens 5 %, mindestens 10 %, mindestens 20 %, mindestens 50 %, mindestens 70 %, mindestens 75 %, mindestens 80 %, mindestens 85 %, mindestens 90 % und/oder mindestens 95 % aller EVs mindestens ein NA-Frachtmolekül umfassen.

11. Verfahren zum Herstellen der EVs nach einem der vorstehenden Ansprüche, umfassend:
(i) Einführen mindestens eines Polynukleotidkonstrukts, das für ein PABP-Protein oder ein Fragment oder eine Domäne davon kodiert, das/die in der Lage ist, an einen zusammenhängenden Abschnitt von Adenosinbasen zu binden, in eine EV-produzierende Zelle; und
(ii) Einführen mindestens eines Polynukleotidkonstrukts, das mindestens ein NA-Frachtmolekül kodiert, in dieselbe EV-produzierende Zelle; und
(iii) Einführen mindestens eines Polynukleotidkonstrukts, das mindestens ein Fusionspolypeptid kodiert, das mindestens eine NA-bindende Domäne und mindestens ein exosomales Polypeptid umfasst, in dieselbe EV-produzierende Zelle; und
(iv) Exprimieren jedes von PABP, dem NA-Frachtmolekül und dem Fusionspolypeptid in der EV-produzierenden Zelle, wodurch die EVs erzeugt werden.

12. Verfahren zum Herstellen von EVs nach Anspruch 18:
(a) weiter umfassend:
(i) Einführen mindestens eines Polynukleotidkonstrukts, das für einen Translationsinitiationsfaktor kodiert, in die EV-produzierende Zelle; und
(ii) Exprimieren des mindestens einen Polypeptidkonstrukts, das durch das Polynukleotidkonstrukt von (i) kodiert wird, in der EV-produzierenden Zelle, wodurch die EVs erzeugt werden;
(b) wobei das PAPB-Protein oder das Fragment oder die Domäne davon einen Teil des Fusionspolypeptids bildet, das mindestens eine NA-bindende Domäne und mindestens ein exosomales Polypeptid umfasst; und/oder
(c) wobei das PABP-Protein oder das Fragment oder die Domäne davon, das NA-Frachtmolekül, das Fusionspolypeptid und/oder der Translationsinitiationsfaktor durch dasselbe oder durch unterschiedliche Polynukleotidkonstrukte kodiert werden können.

13. In-vitro-Verfahren zur intrazellulären Abgabe mindestens eines NA-Frachtmoleküls, umfassend das In-Kontakt-Bringen einer Zielzelle mit mindestens einem EV nach einem der Ansprüche 1-8 und/oder mindestens einer Population von EVs nach einem der Ansprüche 9-10.

14. Pharmazeutische Zusammensetzung, umfassend (i) mindestens ein EV nach einem der Ansprüche 1-8 und/oder (ii) mindestens eine Population von EVs nach einem der Ansprüche 9-10, und einen pharmazeutisch akzeptablen Hilfsstoff oder Träger.

15. (i) Mindestens ein EV nach einem der Ansprüche 1-8, (ii) mindestens eine Population von EVs nach einem der Ansprüche 9-10, und/oder (iii) die pharmazeutische Zusammensetzung nach Anspruch 14, zur Verwendung in der Medizin.

## Revendications

1. Vésicule extracellulaire (EV) comprenant au moins une protéine de liaison à polyA (PABP) ou un fragment ou un domaine de celle-ci capable de se lier à un segment contigu de bases d'adénosine, au moins une molécule cargo d'acide nucléique (NA) comprenant un segment contigu de nucléotides d'adénine et au moins un polypeptide de fusion comprenant au moins un domaine de liaison à un NA et au moins un polypeptide exosomal.

2. EV selon la revendication 1 :
(a) dans laquelle la PABP est comprise dans le polypeptide de fusion ; et/ou
(b) dans laquelle l'au moins un domaine de liaison à un NA est une ou plusieurs protéines de liaison à l'ARNm, protéines de liaison au pré-ARNr, protéines de liaison à l'ARNt, petites protéines de liaison à l'ARN nucléaires ou nucléolaires, protéines de liaison à l'ARN non codantes, protéines de liaison à l'ARNmi, protéines de liaison à l'ARNsh.

3. EV selon l'une quelconque des revendications précédentes, dans laquelle l'au moins une molécule cargo de NA :
(a) est transportée dans l'EV en se liant à la protéine de fusion ;
(b) comprend au moins un site de liaison pour le domaine de liaison à un NA ;
(c) comprend au moins un site de clivage entre le site de liaison à un NA et le domaine du cargo de NA qui a une activité biologique, thérapeutique et/ou prophylactique ;
(d) est sélectionnée dans le groupe comprenant l'ARNsh, l'ARNmi, l'ARNm, l'ARNg, le pri-miARN, le pré-miARN, l'ARN circulaire, l'ARNpi, l'ARNt, l'ARNr, l'ARNsn, l'ARNInc, les ribozymes, l'ADN mini-circulaire et/ou l'ADN plasmidique ; et/ou
(e) est une molécule d'ARNm qui code pour une protéine ou un peptide thérapeutique et/ou prophylactique.

4. EV selon la revendication 3(e), dans laquelle la protéine ou le peptide thérapeutique est choisi dans le groupe comprenant : des anticorps, des intracorps, des fragments variables à chaîne unique, des afficorps, des enzymes, des transporteurs, des suppresseurs de tumeurs, des inhibiteurs viraux ou bactériens, des protéines de composant cellulaire, des protéines de liaison à l'ADN et/ou à l'ARN, des inhibiteurs de réparation de l'ADN, des nucléases, des protéinases, des intégrases, des facteurs de transcription, des facteurs de croissance, des inhibiteurs et des inducteurs de l'apoptose, des toxines, des protéines structurales, des facteurs neurotrophiques, des transporteurs membranaires, des protéines lysosomales, des protéines de liaison aux nucléotides, des protéines de choc thermique, des protéines associées à CRISPR, et tout fragment, domaine et/ou toute combinaison de ceux-ci.

5. EV selon l'une quelconque des revendications précédentes, dans laquelle le polypeptide exosomal est choisi dans le groupe comprenant CD9, CD63, CD81, FLOT1, FLOT2, ALIX, ARRDC1, Syntenin-1, Syntenin-2, Lamp1, Lamp1b, TSG101, Lamp2a, Lamp2b, TSPAN8, syndecan-1, syndecan-2, syndecan-3, syndecan-4, TSPAN14, CD82, CD47, d'autres polypeptides exosomiques et tous fragments de région ou toutes combinaisons de ceux-ci.

6. EV selon l'une quelconque des revendications précédentes, dans laquelle l'EV comprend en outre :
(a) au moins un facteur d'initiation de traduction (TIF) ; et/ou
(b) au moins un groupement de ciblage qui cible l'EV vers une cellule, un tissu, un organe, un organite ou un autre emplacement corporel cible.

7. EV selon l'une quelconque des revendications précédentes, dans laquelle lorsque la molécule cargo de NA est une molécule cargo d'ARNm, elle comprend en outre :
a. une structure secondaire, tertiaire et/ou autre ; et/ou
b. au moins une boucle de tige stabilisatrice ; et/ou
c. au moins un UTR hybride à l'extrémité 5' et/ou 3' .

8. EV selon l'une quelconque des revendications précédentes, dans laquelle l'EV est : un exosome ou une microvésicule.

9. Population d'EV selon l'une quelconque des revendications précédentes.

10. Population d'EV selon la revendication 9, dans laquelle :
(a) le nombre moyen de molécules cargo de NA par EV dans l'ensemble de la population d'EV est supérieur à un par EV ; et/ou
(b) au moins 5 %, au moins 10 %, au moins 20 %, au moins 50 %, au moins 70 %, au moins 75 %, au moins 80 %, au moins 85 %, au moins 90 % et/ou au moins 95 % de toutes les EV comprennent au moins une molécule cargo de NA.

11. Procédé de production des EV selon l'une quelconque des revendications précédentes, comprenant :
(i) l'introduction dans une cellule productrice d'EV d'au moins un produit d'assemblage polynucléotidique codant pour une protéine PABP ou un fragment ou un domaine de celle-ci capable de se lier à un segment contigu de bases d'adénosine ; et
(ii) l'introduction dans la même cellule productrice d'EV d'au moins un produit d'assemblage polynucléotidique codant pour au moins une molécule cargo de NA ; et
(iii) l'introduction dans la même cellule productrice d'EV d'au moins un produit d'assemblage polynucléotidique codant pour au moins un polypeptide de fusion comprenant au moins un domaine de liaison à un NA et au moins un polypeptide exosomal ; et
(iv) l'expression dans ladite cellule productrice d'EV de chacun de la PABP, de la molécule cargo de NA et du polypeptide de fusion, générant ainsi lesdites EV.

12. Procédé de production d'EV selon la revendication 18 :
(a) comprenant en outre :
(i) l'introduction dans la cellule productrice d'EV d'au moins un produit d'assemblage polynucléotidique codant pour un facteur d'initiation de traduction ; et
(ii) l'expression dans la cellule productrice d'EV d'au moins un produit d'assemblage polypeptidique codé par le produit d'assemblage polynucléotidique de (i), générant ainsi lesdites EV ;
(b) dans lequel la protéine PAPB ou un fragment ou domaine de celle-ci fait partie du polypeptide de fusion comprenant au moins un domaine de liaison à un NA et au moins un polypeptide exosomal ; et/ou
(c) dans lequel la protéine PABP ou un fragment ou domaine de celle-ci, la molécule cargo de NA, le polypeptide de fusion et/ou le facteur d'initiation de traduction peuvent être codés par le même ou par des produits d'assemblage polynucléotidiques différents.

13. Procédé *in vitro* pour l'administration intracellulaire d'au moins une molécule cargo de NA, comprenant la mise en contact d'une cellule cible avec au moins une EV selon l'une quelconque des revendications 1 à 8 et/ou au moins une population d'EV selon l'une quelconque des revendications 9 et 10.

14. Composition pharmaceutique comprenant (i) au moins une EV selon l'une quelconque des revendications 1 à 8, et/ou (ii) au moins une population d'EV selon l'une quelconque des revendications 9 et 10, et un excipient ou support pharmaceutiquement acceptable.

15. (i) Au moins une EV selon l'une quelconque des revendications 1 à 8, (ii) au moins une population d'EV selon l'une quelconque des revendications 9 et 10, et/ou (iii) composition pharmaceutique selon la revendication 14 destinée à être utilisé en médecine.
